(19) Europäisches Patentamt
European Patent Office
Office européen des brevets

(11) EP 2 940 146 A1

(12) EUROPEAN PATENT APPLICATION
published in accordance with Art. 153(4) EPC

(43) Date of publication:
04.11.2015 Bulletin 2015/45

(21) Application number: 13868991.4

(22) Date of filing: 26.12.2013

(51) Int Cl.:
*C12P 21/00* (2006.01) *A61L 27/00* (2006.01)
*C07K 14/47* (2006.01) *C07K 14/475* (2006.01)
*C07K 14/65* (2006.01) *C07K 14/78* (2006.01)
*C12N 5/071* (2010.01) *C12N 15/09* (2006.01)

(86) International application number:
PCT/JP2013/084990

(87) International publication number:
WO 2014/104246 (03.07.2014 Gazette 2014/27)

(84) Designated Contracting States:
AL AT BE BG CH CY CZ DE DK EE ES FI FR GB GR HR HU IE IS IT LI LT LU LV MC MK MT NL NO PL PT RO RS SE SI SK SM TR
Designated Extension States:
BA ME

(30) Priority: 26.12.2012 JP 2012282001

(71) Applicant: Quarrymen Corporation
Machida-shi, Tokyo 194-0003 (JP)

(72) Inventor: UEDA, Minoru
Tokyo 108-0075 (JP)

(74) Representative: Kuhnen & Wacker
Patent- und Rechtsanwaltsbüro
Prinz-Ludwig-Straße 40A
85354 Freising (DE)

# (54) HIGHLY FUNCTIONAL IMPLANT MATERIAL

(57) The purpose of the present invention is to provide: a method for preparing a cell product for use in the production of an implant, which can improve the surface properties of a material by a simple technique and can integrate a bone with a metallic implant material satisfactorily within a given period of time; a product produced by the method; and a sophisticated implant produced using the product. A cell product containing Col-I, OCN, OPN, BSP, fibronectin and VEGF or another growth factor which are produced by a bone marrow stromal cell or an immortalized milk teeth stem cell is prepared from the bone marrow stromal cell or the immortalized milk teeth stem cell, and a sophisticated implant having the growth factor immobilized on the surface thereof is provided using the resultant cell product and a mineralizing solution.

EP 2 940 146 A1

## Description

### Technical Field

The present invention relates to a sophisticated implant material using products from bone marrow stromal cells (BMCs) or immortalized dens deciduous stem cells. Specifically, it relates to the sophisticated implant material of which surface is coated with the products from the bone marrow stromal cells or immortalized dens deciduous stem cells.

### Background Art

One of an object of dental treatment is to recover lost functions of teeth and repair a shape of deficient teeth by using artificial materials. Therefore, an implant is used as the most popular treatment to repair the deficient teeth and the like.

Primarily, in order to function the deficient teeth and the like sufficiently with the implant, since the implant should be stabilized, it is desirable that the implant integrate with an alveolar bone. Therefore, bioactive materials such as bioglass, calcium phosphate ceramics (that is, hydroxyapatite or ß-tricalcium phosphate) and the like have been used as these implant materials.

From a point of view that the integration of the implant with a bone, the bioactive materials are superior in terms of their high biocompatibility. On the other hand, from a point of view of the mechanical properties such as strength or durability, metal materials are superior to the bioactive materials, because huge load is applied on the teeth. Then, Titanium (herein below, it is often referred to as "Ti") is used widely among these metal materials because of the biocompatibility, cost and so forth.

As a technique to improve the surface properties of the implant materials, an implant having positively charged metal oxide on its surface, which may pull a cell selected from the group consisting of a human mesenchymal stem cell and osteoblast, or a protein selected from the group consisting of bovine serum albumin, fraction V, and bovine serum fibronectin, is proposed (herein below it is referred as the "prior art 1").

Also, it is known that the integration of the metal implant with the bone depends on the bone reproducibility. Then, in order to improve the integration ability of the implant with the bone, a reagent for osteogenesis or a growth factor is applied onto the surface of the implant material. The past paper (herein below it is referred to as the "prior art 2"), it is reported that the fixed polypeptides or cells on the mucopolysaccharide-coated implant improve the osseointegration and the bone recovery after the implantation.

### Prior Art

[Patent Document]

[Patent Document 1] JP2012-509750A
[Patent Document 2] JP2004-531461A

### Summary of the Invention

### Problems to be solved by the Invention

The prior art mentioned above is superior in the point of view that it has progressed integration of the implant with the bone, compared to that of the implant having untreated material surface. However, there was a problem that it has insufficient integration of the implant with the bone, compared to that of the implant made of bioactive material with high biocompatibility.

Also, the material surface of the metal implant is pre-treated for activating the metal surface firstly; then it is treated with osteogenesis agent or growth factors application. Therefore, the treatment procedure is very complicated. Furthermore, the maintained period for the growth factor fixed on the surface becomes problem, when the growth factor is applied onto the material surface of the metal implant. Because, the sufficient integration cannot be desirable, if the period to maintain the growth factor on the material surface of the metal implant is short compared to that to maintain the bone and the metal implant material are integrated mutually.

Therefore, there is a strong need to develop the surface properties of the material by using a simple procedure to integrate the material with the bone sufficiently in a certain period.

## Means for solving the problem

The present invention was completed under the situations as mentioned above.

Namely, the first aspect of the present invention is a preparation method for cell product comprising the steps of: preliminary culturing 5 x $10^3$ to 5 x $10^4$ cell/mL of the bone marrow stromal cells or immortalized dens deciduous stem cells in a medium containing 5 to 15% serum, 50 to 150 U/mL of penicillin and 50 to 150 μg/mL of streptomycin at a temperature from 34 to 37.5 °C in the presence of 5% $CO_2$; selecting the cell having both of a prescribed shape and adhesive property; and culturing the cells selected in said selecting step under the same condition as that of the preliminary culturing to collect a culture supernatant after 40 to 56 hours from start of the culture.

Here, said bone marrow stromal cell is preferably derived from a rat femur bone; the immortalized dens deciduous stem cells is preferably derived from any one of the cells selected from the group consisting of a swine dental pulp and a human dens deciduous. Said cell is preferably incubated at 37°C.

Also, said prescribed form in the step of selecting cell is preferably a spindle shape and a fusiform. Said culture supernatant is preferably collected after 44 to 52 hours from the start of the culture, and more preferably collected after 48 hours from the start of the culture.

Here, said culture medium preferably comprises at least one of the medium selected from the group comprising of Dulbecco's medium, Iscove's modified Dulbecco's Medium, Ham's F12 medium and RPMI1640. Also, said serum is preferably at least one of the medium selected from the group consisting of fetal bovine serum, bovine serum, horse serum, human serum, and sheep serum. Furthermore, said culture supernatant preferably comprises 10% fetal bovine serum, 100 U/mL of penicillin and 100 μg/mL of streptomycin.

Also, the above-preparation method preferably further comprising the step of; preparing a precipitate by performing centrifugation and ethanol precipitation treatment; and lyophilizing the precipitate obtained from said preparation step of precipitate.

The second aspect of the present invention is the cell product prepared by using the above-mentioned preparation method. Said culture supernatant preferably comprises at least type I collagen (Col-I), fibronectin, decorin and vascular endothelial growth factor (VEGF). In addition to these cell products, said culture supernatant preferably further comprises insulin-like growth factor - binding protein 7, follistatin related protein, metalloproteinase inhibitor I, tissue plasminogen activator inhibitor, actin, and sulfhydryl oxidase I, SPARC, collagen α2 (IV) chain, β-2-microglobulin and Rho GTPase-activating protein 18.

Also, the present invention is production method for sophisticated implant comprising the steps of: washing a surface of an implant material with an organic solvent to prepare a washed implant material; immersing said washed implant material in a first calcification solution having prescribed composition at prescribed temperature for 3 to 6 hours to prepare an immersed implant material; and incubating said immersed implant material in a second calcification solution containing the cell product at a prescribed concentration according to any of the claims 12 to 14 for 2 to 5 days

Here, said implant material is preferably formed by any material selected from the group consisting of titanium, zirconia, hydroxyapatite and β-TCP. Also, said organic solvent is preferably composed of acetone and 60 to 80% of ethanol. Furthermore, said first calcification solution is preferably composed of 100 to 150 mM of NaCl, 2.5 to 3.5 mM of $CaCl_2 \cdot H_2O$, 1.5 to 2.5 mM of $K_2HPO_4$ and 25 to 75 mM of Tris-HCl (pH 7.2 to 7.6). More preferably, it is composed of 136.8 mM of NaCl, 3.10 mM of $CaCl_2 \cdot H_2O$, 1.86 mM of $K_2HPO_4$, and 50 mM of Tris-HCl (pH 7.4).

Furthermore, said prescribed temperature is preferably at 36 to 38 °C, and immersing time is preferably for 4 hours. Said cell product of prescribed concentration is preferably at a concentration of 5 to 20 mg/mL, and more preferably about 10 mg/mL.

The third aspect of the present invention is sophisticated implant produced by the method described above. Here, said sophisticated implant is preferably formed by a material selected from the group consisting of titanium, zirconia, hydroxyapatite and β-TCP. Said implant preferably has at least type I collagen (herein below, it is referred to as Col-I), fibronectin and decorin on the surface. In addition to these them, said implant preferably further comprises at least one of protein selected from the group consisting of insulin-like growth factor - binding protein 7(herein below, it is referred to as IGF-binding protein 7), follistatin related protein, metalloproteinase inhibitor I, tissue plasminogen activator inhibitor, actin and sulfhydryl oxidase I on the surface of the implant.

## Advantageous Effect of the Invention

According to the present invention, the cell product containing Col-I, decorin, vimentin, IGF-binding protein 7, fibronectin, SPARC (secreted protein acidic and rich cysteine) and the like obtained from the bone marrow stem cell and immortalized dens deciduous stem cell is prepared. Also, the sophisticated implant is prepared easily by this cell product.

Furthermore, the sophisticated implant material which integrates with bone in a short period is obtained.

**Brief Description of Drawings**

Fig. 1 is an electron micrograph of the surface of the Ti implant by using a scanning electron microscope. In Fig. 1, (A) and (D) show the surfaces treated with PBS (phosphate buffer saline), (B) and (E) show these treated with DMEM (Dulbecco's Modified Eagle's Medium), (C) and (F) show these treated with CM (the culture supernatant of the cell culture).

Fig. 2 is a graph showing cell numbers adhered on the surface of the Ti implant treated as mentioned above (herein below, it is sometimes referred to as "Ti plate") after 24 hours from seeding 1.0 x $10^6$ cells on the Ti plate (ANOVA test. In the figure, * shows $p < 0.05$.).

Fig. 3 shows a gel electrophoresis image of BMSCs cultured on the Ti-plate surface having fixed DMEM or CM in (A) to (C), and a graph of the quantitatively determination of them in (D) to (F).

Fig. 4 is a detection image of biomolecules labeled with QD on the Ti-plate in vivo. In the figure, PBS, DMEM and CM are the same as described above.

Fig. 5 is the graph showing a relationship between population doubling number and culturing times of immortalized stem cell selected from the dental pulp cell as described above or that of non-immortalized stem cell. In Fig. 1, SHED-T shows the immortalized stem cell, and SHED-C shows the non- immortalized stem cell.

Fig. 6 is the graph showing STRO-1 expression results in both SHED-C and SHED-T (see, Figs. 2(A) to (D)). Wherein, PD20 shows that the population doubling time is 20, PD30 shows that it is 30, and PD40 shows that it is 40.

Fig. 7 is the tissue staining images of SHED-C or SHED-T at the population doubling time shown in Fig. 8.

Fig. 8 is the graph showing the relationship between the population doubling time (number of times) and mass of neonatal bone. In the figure, ** shows $p < 0.05$, *** shows $p < 0.01$. The mass of the neonatal bone is calculated by using the following equation.

The mass of the neonatal bone = area of the neonatal bone / sight area x 100

Fig. 9 is the graph showing the value of torque removal of Ti-implant into the rat femur (N=3), which was observed for 28 days in PBS, DMEM or CM (ANOVA test. * shows $p < 0.05$, ** shows $p < 0.01$).

Fig. 10 is the graph showing the change of Bone-to-implant contact (Bone-to-implant contact, herein below, it is referred to as BIC) in a cancellous bone or cortical bone of the femur (N=3). (A) shows that in the cancellous bone, and (B) shows that in the cortical bone. In the figure, *shows $p < 0.05$, ** shows $p < 0.01$, as the result of ANOVA test.

Fig. 11 is the graph showing the time-dependent change of a stability of the implant treated with the cell product of the present invention or without that (N=3). In the figure, *shows $p < 0.05$, as a result of ANOVA test.

Fig. 12 is the graph showing differences of the cell numbers treated with PBS or the cell product of the present invention. In the figure, P shows the case treated with plasma and N shows not treated under atmospheric pressure. *shows $p < 0.05$, ** shows $p < 0.01$, as the result of ANOVA test.

Fig. 13 shows an observation result of the cell growth, stained with DAPI (4', 6-diamine-2-phenylindol) and phalloidin by using a fluorescent microscope in the case of Fig. 12 (N=3). In the figure, scale bar shows 100 $\mu$m of length.

Fig. 14 shows the change of the growth ratio of the cell, when the cell product of the present invention was stored at 4°C, -15°C, -80°C for 1 week, 2 weeks or 1 month respectively, and then the same amount of them was separately added in the medium (N=3).

**Mode for Carrying Out the Invention**

The present invention is explained in detail in below.

A preparation method of the present invention comprises the steps of: (a) preliminary culturing 5 x $10^3$ to 5 x $10^4$ cell/mL of the bone marrow stromal cells or immortalized dens deciduous stem cells in a medium containing 5 to 15% serum, 50 to 150 U/mL of penicillin and 50 to 150 $\mu$g/mL of streptomycin at a temperature from 34 to 37.5 °C in the presence of 5% $CO_2$; (b) selecting the cell having both of a prescribed shape and adhesive property; and (c) culturing the cells selected in said selecting step under the same condition as that of the preliminary culturing to collect a culture supernatant after 40 to 56 hours from start of the culture.

Here, said culture medium comprises at least one of the medium selected from the group comprising of Dulbecco's medium (herein below it is often referred to as "DMEM"), Iscove's modified Dulbecco's Medium (herein below it is often referred to as "IMDM"), Ham's F12 medium (herein below it is often referred to as "Ham12") and RPMI1640, because they are easily obtained and handled. The medium is so called a basal medium, and may be purchased from GIBCO, SIGMA and so on. Among them, two or more medium may be concomitantly used as a combined medium. An example of the mixed medium, there are mentioned such as a medium including IMDM and HamF12 in an equal amounts (for example, it is commercially available as the product name, IMDM/HamF12 from GIBCO).

Also, the serum is preferably at least one of selected from the group consisting of fetal bovine serum, bovine serum, horse serum, human serum, and sheep serum; because they promote the cell growth without any controversial protein production by the cells into the culture supernatant.

As components to be supplemented to the medium, besides the above serum, there are mentioned, for example, a serum replacement (such as Knockout serum replacement, and the like), bovine serum albumin (BSA), antibiotics such as penicillin, streptomycin, gentamicin and the like, vitamins such as vitamin K, folic acid and the like, minerals such as calcium, magnesium and the like, amino acids such as arginine, tryptophan and the like.

In the preparation of the present invention, it is preferable to use the medium including 10% of fetal bovine serum, 100 U/mL penicillin and 100 μg /mL of streptomycin to obtain the culture supernatant containing a sufficient amount of the cell growth factors.

Also, the present invention may be the method further comprising the steps of: (d) preparing the precipitate by using a centrifugation and ethanol precipitation; and (e) lyophilizing the precipitate obtained in the preparation step of precipitate.

The bone marrow stromal cell is preferably derived from any one of cells selected from the group consisting of the rat femur, the swine lower jaw, the swine tooth, and the human dens deciduous, because the cells, which may constantly produce a certain kinds of the cell growth factors, may be reliably obtained.

Here, the cell proliferation factor is general term for the polypeptide factors related to a specific cell growth, differentiation and the like, including immuno-modulating lymphokines or cytokines. It is also referred to as growth factor or cell growth factor. Herein below, the cell proliferation factor is sometimes simply referred to as the "growth factor".

It is known that the growth factor becomes involved in a modulation of a variety of cytological or physiological process in vivo. The growth factor binds specifically receptor proteins on the surface of the target cell (herein below, the receptor protein is simply referred to as the "receptor" or "receptor") to perform signaling.

As typical growth factors, there are mentioned such as epidermal growth factor (Epidermal growth factor: EGF), insulin-like growth factor (Isulin-like growth factor: IGF), transforming growth factor (Transforming growth factor : TGF), nerve growth factor (Nerve growth factor: NGF), brain-derived neurotrophic factor (Brain-derived neurotrophic factor: BDNF), vesicular endothelial growth factor (Vesicular endothelial growth factor: VEGF), granulocyte-colony stimulating factor (Granulocyte-colony stimulating factor: G-CSF), granulocyte-macrophage-colony stimulating factor (Granulocyte-macrophage-colony stimulating factor: GM-CSF), platelet-derived growth factor (Platelet-derived growth factor: PDGF), erythropoietin (Erythropoietin: EPO), thrombopoietin (thrombopoietin: TPO), basic fibroblast growth factor (basic fibroblast growth factor: bFGF or FGF2), hepatocyte growth factor (Hepatocyte growth factor: HGF) and the like.

TGF-β is one of the growth factor presented in nature. It plays extremely important roles in histogenesis, cell differentiation and embryo development as the same as other factors through many other signal paths.

B type transforming growth factor, TGF-β, is produced in the almost all of cells such as kidney, bone marrow, platelet and the like. It has five subtypes (β1 to β5). TGF-β1 to β3 are accumulated in the bone matrix as inactive forms and activated by acid released by osteoclast in bone resorption. TGF-β promotes the osteoblast growth and synthesis or growth of the mesenchymal cell such as collagen. However, it rather suppresses the epidermal cell growth and osteoclast.

In the receptor having tyrosine kinase activity, the binding of the growth factor makes tyrosine residue phosphorylation to cause the cell growth and differentiation. Also, there is an example that the growth factor becomes the mesoderm inducer in ontogenesis.

The growth factors are classified into several relating families in the aspect of their structure and evolution. As these families, there are mentioned such as TGF-β, bone morphogenic protein (bone morphogenic protein: BMP), neurotrophin (neurotrophic factor), fibroblast growth factor (FGF) and the like. As the neurotrophin, there are mentioned such as NGF, BDNF, NT3 and the like. The growth factor is frequently used for medical treatment currently.

Also, TGF-β is one of the members constituting the TGF-β superfamily. In the superfamily, bone morphogenic protein (bone morphogenic protein: BMP) and other proteins play an important role in the osteogenesis of a living body are included.

In the culture supernatant of the present invention, the following proteins are preferably comprised: collagen α-1(I) chain, collagen α-2(I) chain, vimentin, collagen α-1(IV) chain, IGF binding protein 7 (IGFBP7), fibronectin, decorin, plasminogen activator inhibitor 1, actin (cytoplasmic 2), sulfhydryl oxidase 1, SPARC, metalloproteinase inhibitor 1, collagen α-2(IV) chain, SCP2, 72 kDa type IV collagenase, β-2 microglobulin, Rho GTPase activating protein 18.

Here, it is known that collagen is a molecule having triple helical structure composed of three α chains, and that it is classified into many types such as type I, type II, type III, type IV and Type V. It is known that the polypeptide (α chain) is classified into more than 30 species, and they are referred to as, for example, α-1(I), α-2(IV), and the like.

The collagen protein family is classified into as follows:

1) a group including a molecule which forms filaments having periodic and striated structure of 67 nm (type I, type III, type V (in the tissue except cartilage mainly), type II and type XI (in the cartilage tissue)

2) a group including the molecule which binds on the surface of these filament (type XII, type IV (in the tissue except cartilage) and type IX (in the cartilage tissue)

3) a group including the molecule which forms web of associate (mainly type IV collagen forming a skeleton of the basement membrane), type VI forming the extracellular fine filament, and type VII forming anchoring fibril penetrating basement lamina from epidermal basal cells in the skin and the like.

4) type X which forms hexagonal lattice (it exists tentatively in the period when a cartilage changes a bone, it forms the hexagonal structure of the Descemet's membrane in cornea and it exists in the blood vessel and other tissues), type XIII, type XV, type XVII and type XVIII which have a domain penetrating the cell membrane.

The vimentin is an intermediate filament protein which characteristically expresses in the mesenchymal cell such as fibroblast and leukocyte. The construct of the vimentin filament is controlled under phosphorylation by various protein kinases. It is known that vimentin expresses transiently in the course of development.

Insulin-like growth factor-binding protein 7 (IGFBP7) binds IGF-1 receptor and block the activation of the IGF-1 receptor by insulin-like growth factor. Therefore, the abundance of IGFBP7 shows inversely relation to tumor progression.

Fibronectin is a glycoprotein that forms extracellular matrix, and dimer of the peptide having a molecular weight of about 250 kDa. It mainly promotes adhesion of fibroblast, hepatocyte, nerve cell and the like. It affects cell migration, phagocytosis and the like besides the cell adhesion through integrin which is specific receptor on the surface of the cell membrane, and also behaves in the site of the wounded tissue.

A decorin is a proteoglycan which has a hybrid chain of a chondroitin sulfate in the small type of dermatan sulfate proteoglycan, bone proteoglycan type II, PG-40, PG-II, PG-2, DS-PGII and the core protein having 38 kDa (the molecular weight of $3.8 \times 10^4$), which exists binding a collagen fiber. It distributes widely in the connective tissue of the animal such as cartilage, bone, tendon, skin, sclera, aorta and the like.

Plasminogen activator inhibitor (PAI) binds plasminogen activator of tissue which plays an important role in lysis of fibrin, and inhibits its function. The most important PAI is PAI-1 produced in the vascular endothelial cell, and it exists also in the platelet. Besides this, there are PAI-2 produced in the placenta and PAI-3 which inhibits active protein C (APC). An increased blood PAI-1 level causes thrombotic tendency. PAI-1 is a kind of acute phase proteins, which level is higher in afternoon compared to morning, and is positively correlated with that of blood triacylglycerol.

Actin (cytosolic type 2) is a protein to contract a muscle. It is thought that action mainly functions as actin filament in the cell. In a muscular cell, actin filament assembles with type II myosin to form a fascicle of actomyosin and contribute to muscular contraction power. In the non-muscular cell containing the nerve cell, the actin filament forms a variety of actin conformation such as filopodia having protrusion portion (Filopodia, filopodia), lamellipodia having the ruffling structure of the cell periphery (Lamellipodia, lamellipodia), and a stress fiber being composed of the fascicle of actomyosin associated with activated type II myosin.

The reorganization of the actin structure is controlled under the intracellular signaling spatiotemporal specifically through low molecular weight G-protein belonging to Rho family. It plays the important role in the dynamic control of cell morphology such as cell movement or cell division. Simultaneously, the reorganization contributes to the vesicle transport along the actin filament through the binding with the atypical myosin. In addition to these dynamic roles, the actin filament accumulates under the immediately beneath the cell membrane to form the undercoat of cell-cortex (cell cortex), or supports the intercellular adhesion by binding with the cadherin, which is the cell adhesion molecules, through $\alpha$-catenin or vinculin.

Sulfhydryl oxidase 1 is a thioloxidase, of which substrate is R'C(R)SH.

SPARC is a non-collagen protein of 30 kDa existing in the bone and dentin, and plays a key role in the function of the dermis formation. It is secreted from the fibroblast and enhances both of the synthesis of collagen and hyaluronic acid.

Metalloproteinases inhibitor (TIMP, tissue inhibitors of metalloproteinase) is an endogenous inhibitor which controls the activity of matrix metalloproteinase (matrix metalloproteinase: MMP). MMP is belonging to zinc metalloproteinase family, and controls the metabolism of extracellular matrix secreted from the cells. At present, it is known that there are TIMP and TINP1 to 4, which have the different inhibition pattern respectively, and express at different level in every tissue depending on the inflow of MMP. TIMP-1 and -2 bind to many active site of MMP and inhibit their activity irreversibly. Also, these two TIMP affect a variety of cell directly to show growth-stimulating activity.

SCP2 is a microbe body or a protein extract from the body which is produced in large amounts from the fermentation feedstock such as petroleum, natural gases, alcohols, blackstrap molasses and agriproducts. It is also referred to as a "monocellular protein". It is sometimes indicated as a dry matter produced by sterilizing protein-rich unicellular organism such as yeast, bacteria, filamentous bacterium and algae and the like to dry.

Collagenase type IV of 72 kDa is referred to as gelatinase. Among gelatinase, A enzyme is referred to as collagenase type IV of 72 kDa (MMP-2). B enzyme of 95 kDa is referred to as MMP-9. MMP-2 has three repeat sequences of type II fibronectin domain, which is inserted into the catalytic domain, belonging to gelatinase group of MMPs together with MMP-9. MMP-2 indicates substrate specificity directly or indirectly to collagen I, IV, V, VII, X, XIV, gelatin (gelatin), elastin, laminin-1, laminin-5, myelin basic protein (myelin basic protein), MMP-1, MMP-9 and MMP-13.

β-2-microglobrin is a light chain in the double strand configuring the histocompatibility antigen (class I), but it lacks antigenicity to show the specificity of the histocompatibility antigen.

Rho-GTPase activator protein is a group of G-proteins without subunit structures of 20,000 to 30,000 of molecular weight, constituting the superfamily. This superfamily is classified into five groups, Ras, Rho, Rab, Arf, and Ran. Among them, Rho-GTPase activator protein belongs to Rho. Rho protein controls the contraction of smooth muscle, cellular division, cellular movement, and cell morphology through reorganization of actin filament.

A bone marrow stromal cell is referred to as the cell that exists in the bone marrow, easily taken with a bone puncture, to support hematogenesis. Here, in the bone marrow, the cells classified roughly into two types, one of which is blood cell and the other is stroma cell supporting the blood cell, are included, and the bone marrow stromal cell is included in the latter cell. The blood cell proliferates in suspension when they are cultured; in contrast, the bone marrow stromal cell proliferates in adhesion to the wall when they are cultured. The shape of the bone marrow stromal cell is the same as that of the mesenchymal cell; however, it forms the reticulum structure in the bone marrow. The cell may be cultivated as the case that of general fibroblast. Since the stromal cell derived from mesenchymal cell may differentiate into various cells. The bone marrow stromal cells are differentiated to be induced to osseous cell, cartilage cell, adipose cell and skeletal muscle cell.

A dental pulp cell is a kind of stem cell included in the dental nerve. It is said that the dental pulp cell is covered by tooth made from hard material, which is lightproof against ultraviolet ray or radioactive ray so that the gene included in it is not easily wounded.

An immortalized dental pulp stem cell is the immortalized cell of dental pulp stem cell derived from dens deciduous. The dental pulp stem cell is a kind of stem cell contained in the nerve of the tooth. It is said that the dental pulp cell is covered by tooth made from hard material, which is lightproof against ultraviolet ray or radioactive ray so that the gene included in it is not easily wounded.

The immortalized stem cell is produced as follows. At first, an exfoliate dens deciduous is sterilized with antiseptic solution such as chlorhexidine, Isodine and others. After that, if the femur of the animal is used as a source, the bone marrow cell is taken out by using the syringe, which is filled with PBS or the like, with injection needle of which outside diameter tightly matches with the medullar cavity of the femur, so as to extrude the bone marrow cells from the medullar cavity. Also, when the swine tooth or human exfoliate dens deciduous are used as the cell source, the dental crown is divided respectively, and collect the dental pulp tissue with dental reamer.

The obtained bone marrow stromal cells or dental pulp tissues are suspended in the basal medium, for example, Dulbecco's Modified Eagle's Medium (herein below, it is referred to as "DMEM") containing 5 to 15 % of calf serum (herein below, it is sometimes referred to as "CS") and 50 to 150 unit/ml of antibiotics. After that, the cells are treated with 1 to 5 mg/ml of collagenase and 1 to 5 mg/ml of dispase at 37 °C for 0.5 to 2 hours.

As the basal medium, there are mentioned, for example, DMEM; it is used with fetal bovine serum is supplemented, and after treatment with an enzyme, the medium is centrifuged (3,000 to 7,000 rpm) for 3 to 10 minutes to collect the dental pulp cells. As needed, the cells are sorted by using a cell strainer. The sorted cells are resuspended with 3 to 6 ml of the basal medium, and are plated in the dish for adherent cell culture, of which dish having 4 to 8 cm in diameter.

The medium, for example, DMEM containing 10% FCS is added to the dish, then, it is incubated in the incubator with 5% $CO_2$ at 37 °C for about 2 weeks, for example, while exchanging the medium every few days. During the incubation, passage is performed 2 to 4 times. After confirming the cell becomes sub-confluent, the medium is removed and the cells are washed with PBS and the like from 1 time to several times. Instead of removing the medium and washing the cells, adherent dental pulp stem cells formed colonies may be collected. The adherent dental pulp stem cells are treated, for example, with 0.025 to 0.1% trypsin and 0.3 to 1 mM of EDTA at 37 °C for several minutes to release. Then, the cells are collected.

In the case of the dental pulp stem cell, after treating with the enzyme, the cells are centrifuged (3,000 to 7,000 rpm) for 3 to 10 minutes to be recovered. As needed, the cells are sorted with the cell strainer. The sorted cells are resuspended in the 3 to 6 ml of the basal medium, and plated into the dish with 4 to 8 cm in diameter for adherent cell culture.

After that, the medium, for example, DMEM containing 10% FCS is added into the dish for the desired periods as mentioned above. The cells are washed with PBS and the like for 1 time to several times to obtain the adherent cells.

In the case of using the rat bone marrow cells, it is preferable that a bone fluid obtained from the marrow space of the rat femur (about 30 to 100 μg/femur) is added into DMEM, which contains about 5 to 15% fetal bovine serum (FBS), penicillin and streptomycin (provided from Wako Pure Chemical Industries, Ltd.) to be incubated in the incubator with 5% $CO_2$ at 37 °C by 2 to 4 passages of the preliminary incubation to select the cells having desired properties.

For example, the bone fluid (about 50 μL/femur) is added into DMEM including 10% FBS, penicillin and streptomycin of the above mentioned concentrations in the incubator with 5% $CO_2$ at 37 °C through 3 passages of the preliminary incubation to select the cells having the spindle shape, adherent properties and the like.

As mentioned above, the sorted adherent cells (the stem cells) are obtained. After that, for example, the stem cells are plated into the dish for adherent cell culture to be incubated under the condition of 5% $CO_2$ at 37 °C.

When the BMSCs obtained from the femur are used, the cells having the spindle shape and adherent properties are solely selected and used.

In passage culture, for example, the cells are collected by using trypsin and EDTA as mentioned above, when the cells become sub-confluent or confluent with macroscopic observation. Then, the cells are plated again in the culture dish including the culture medium.

Here, the term, "sub-confluent", means the situation that the cells adhere about 70 % of the area of the culture vessel on which the cells should be adhered. For example, the passage is performed from 1 time to 8 times to select the cells, which are grown up to achieve the necessary cell number, for example, about 1 x $10^7$ cells/mL. After culturing as described above, the cells are collected to store in liquid nitrogen. The cells collected from a variety of donors may be stored in the form of dental pulp stem cell bank.

When the immortalized stem cells are prepared, primarily cultured cells are obtained through primary culture of the stem cell obtained as mentioned above. Genes, hTERT, bmi-1, E6 and E7, are introduced into the primarily cultured cell as follows. A plasmid to introduce these genes is prepared, and it is integrated into a shuttle vector such as pShuttle2 for cloning the genes. E.coli is transformed by using the shuttle vector to select kanamycin-resistant transformant. The plasmid DNA in the kanamycin-resistant transformant is purified and the recombinant is identified by analyzing restriction sites.

Next, restriction enzymes, for example, PI-Sce I and I-Cue I are used to cut out an expression cassette from the shuttle vector for ligating it into adenovirus vector, for example, Adeno-X viral DNA. Obtained ligation product is cleaved by using Swa I, and it is used to transform the E. coli.

Here, hTERT is the gene for telomere repair enzyme; bmi-1 is the gene of Bmi-1, which is one of proteins being composed of polycomb group complex. Here, Bmi-1 is necessary for maintaining the hematopoietic stem cells with the action to increase the stem cell number by enhancing the activities.

Both E6 and E7 are early genes of either HPV-16 or HPV-18. Also, Oct3/4 is the gene that cooperates with Sox2 to activate the transcription of the target gene. Klf4 (Kruppel type transcription factor 4) modulates the genes relating to the cell division and the embryogenesis, and it relates to the gastrointestinal system cancer as the tumor inhibition factor.

Sox2 is belonging to SRY-related HMG box gene family, and it is known as the gene got involved in the maintenance of undifferentiated functions of the cells (pluripotency). c-Myc is an oncogenic gene, and it promotes both of survival and death of the cell in the c-Myc-induced tumor. p16INK4a is the gene which plays important role to control the cell cycle of the tumor cell.

Among the transformant obtained, the ampicillin resistant transformants are selected. The recombinant adenovirus DNA, in which the genes as mentioned above are integrated, is purified to identify the recombinant by analyzing the restriction enzyme sites.

After that, the recombinant adenovirus is digested by using Pac-I to be transfected into HEK293 cells. The recombinant adenovirus is grown, and then they are collected to determine the titer of them. According to the conventional method, the virus is purified. Then, they are passed to SHED as target cell.

The virus infected cells are stained by using FITC according to the common procedure to detect STRO-1 positive cell by using a flow cytometer. Here, STRO-1 is thought as one of makers for the mesenchymal stem cell having the pluripotency in the bone marrow, and it becomes an index of immortalization of the cell.

According to the procedure stated above, the bone marrow stromal cell, the dental pulp cell and the immortalized dens deciduous stem cell derived from dental pulp are obtained.

It is preferable to use the bone marrow stromal cell, the dental pulp cell and the immortalized dens deciduous stem cell derived from rat, swine or human dens deciduous, because the source for these cell are available easily and the cells stably producing the growth factor as mentioned above are obtained. Also, these cells are preferably incubated at 37 °C in the preliminary culture step of (a).

Also, the cells having the spindle shape, fusiform, and adherence property are preferably sorted in the cell sorting step. Moreover, it is preferable to collect the culture supernatant after 44 to 52 hours from the incubation start, because the cells having the spindle shape, fusiform and the adherence property are abundantly contained in them. Also, it is more preferable to collect the culture supernatant after 48 hours from the incubation start, because the cells having higher adherence property are obtained.

The culture supernatant preferably contains at least type I collagen, fibronectin, decorin, vascular endothelial cell growth factor (VEGF) and bone sialoprotein (BSP), in which these proteins are produced by the sorted cells, because the integration of the metal implant material and the bone is promoted. The culture supernatant preferably further contains at least one of protein selected from the group consisting of insulin-like growth factor binding protein 7, follistatin-related protein, metalloproteinase inhibitor I, tissue plasminogen activator inhibitor, actin, sulfhydryl oxidase I, insulin-like growth factor (IGF)-1, hepatocyte growth factor (HGF), and TGF-$\beta$, because the integration of the metal implant material and bone is further promoted.

Next, at the preparation step of the precipitate, the culture supernatant as mentioned above is centrifuged to subject to ethanol precipitation to prepare the precipitate. For example, the cells are centrifuged from 1,000 to 2,000 rpm for 3 to 10 minutes, from 2,500 to 3,500 rpm for 1 to 5 minutes, in the range from 0 to 10 °C to remove unnecessary cells. After that, about 8 to 12 times volume of ethanol is added to the centrifuged supernatant, and then it is incubated at -5 to 15 °C for 30 to 90 minutes. Subsequently, the supernatant is centrifuged at from 10,000 to 20,000 for 10 to 20 minutes at from 2 to 6 °C to obtain the precipitate by removing the supernatant. After that, the precipitate obtained by the precipitate preparing step is lyophilized. For example, the precipitate is resuspended in 80 to 95% cold ethanol to centrifuge at from 10,000 to 20,000 rpm for 10 to 20 minutes at from 2 to 6 °C to remove the supernatant. The precipitate is frozen at from -60 to 90 °C to be lyophilized.

According to the above method, the product of the cell as mentioned above is obtained. The obtained cell product is preferably preserved at from -20 to -40 °C, from the view point of the retention of the activity.

Then, the sophisticated implant is produced by using the lyophilized product obtained as mentioned above as follows. Specifically, it is produced by using the method comprising the steps of (f) washing the surface of the implant material by using an organic solvent to be washed implant material, (g) immersing said washed implant material in calcification solution having predetermined composition at predetermined temperature for 3 to 6 hours to be immersed implant material, and (h) treating said immersed implant material by using the in the solution comprising the cell product with predetermined concentration.

Here, it is preferable that said implant material is manufactured by any one of the material selected from the group consisting of titanium, zirconia, hydroxyapatite, and β-TCP, because they have excellent compatibility to the biological tissue and processability, and they may be easily obtained. Among them, it is more preferable to use titanium (Ti), because it is easily processed by using the culture supernatant of the stem cell, and highly efficient.

Also, acetone and from 60 to 80% ethanol (v/v) is preferably used as the organic solvent, because it shows high washing effect to the implant material surface. Also, the ethanol content is set to 60 to 80 %, because the organic solvent has weak detergency when the ethanol content is less than 60%, and higher washing effect is not shown over 80 %. About 70% ethanol is preferably used.

Next, the implant material washed as described above is immersed in the calcification solution having the predetermined composition for 3 to 6 hour. It is preferable that the composition of the first calcification solution contains 100 to 150 mM of NaCl, 2.5 to 3.5 mM of $CaCl_2 \cdot H_2O$, 1.5 to 2.5 mM of $K_2HPO_4$, 25 to 75 mM of Tris-HCl (pH 7.2 to 7.6), from the view point of the calcification efficiency. It is further preferable that the solution contains 136.8 mM NaCl, 3.10 mM $CaCl_2 \cdot H_2O$, 1.86 mM $K_2HPO_4$, 50 mM Tris-HCl (pH 7.4), because the calcification efficiency is high.

Here, the immersing time period is set to 3 to 6 hours, because the calcification effect is insufficient less than 3 hours, and no further calcification effect is obtained over 6 hours. It is preferable that the immersing time period set to 4 hours, because of sufficient calcification.

Also, the immersion is preferably performed at from 36 to 38 °C, because it becomes priming for the immersion in the second calcification solution described in below. It is more preferable that the immersion is performed at 37 °C.

The implant material, which is subjected to the immersion treatment as mentioned above, is incubated in the second calcification solution containing the culture at the predetermined concentration for the predetermined time period; thereby Col-I, BSP, VEGF and other growth factors are attached on the implant material. The second calcification solution preferably contains 130 to 160 mM of $KNO_3$, 1 to 2 mM of $Ca(NO_3)_2 \cdot 4H_2O$, 0.5 to 1.5 mM of $K_2HPO_4$ (pH 7.0 to 7.8), because these proteins attach highly efficiently.

Specifically, the implant material is preferably incubated in in the solution containing 142.8 mM of $KNO_3$, 1.5 mM of $Ca(NO_3)_2 \cdot 4H_2O$ and 0.9 mM of $K_2HPO_4$ (pH 7.4), including 5 to 20 mg/ ml of lyophilized cell culture (cell products) obtained as described above, at room temperature for 2 to 5 days; because the sufficient amounts of the growth factors are attached on the surface of the implant. It is more preferable that the incubation time is for 3 days, because the sophisticated implant is efficiently produced.

Here, the solution having the concentration of the product less than 5 mg/mL causes the problem that the amounts of the growth factor attached on the implant is insufficient and they gives weak adhesion property with the alveolar bone. On the other hand, the solution having the concentration over 20 mg/mL does not give much higher adhesion property. It is more preferable that the implant material is treated by using the solution including the concentration at 10 mg/ml of them to give sufficient adhesion to alveolar bone.

As described above, the sophisticated implant is obtained having at least Col-I, bone sialoprotein (BSP) and VEGF on its surface. When the implant further comprises IGF-1, HGF and TGF-β on its surface, it gives highly improved stability; because they promote the integration of implant with the in a short time.

## Example

The present invention is explained in detail by using the examples. However, the present invention is not limited to the following examples.

**(Example 1)**

(1) Material and method

(1-1) Animal

Female 6 weeks of SD rats (n = 15, 200 to 230 g of body weight) were purchased from Japan SLC, Inc. All experiments were performed according to the experimental guideline of the school of medicine of Nagoya University. These animals are bred at room temperature under 12 hour light-dark cycle. These rats had been given feed and drink water freely.

(1-2) Cell culture

Rat BMSCs were obtained from marrow space of rat femurs. The bone fluid (50 μg/femur) was collected, and then it was added into Dulbecco's Modified Eagle Medium (DMEM, SIGMA-ALDRICH) containing 10% FBS (Wako pure chemical industries, Ltd.), penicillin and streptomycin (Wako pure chemical industries, Ltd.). Then it was subjected to preliminary culture for 3 passages in the 5% $CO_2$ incubator at 37 °C. The medium was changed every other day. The cells having the spindle shape and fusiform, adhesive property and the like were only used.

(1-3) Preparation of CM

Cultured BMSC was grown by the time they became 70 to 80% of confluent. Then, they were washed with warmed phosphate buffered-saline (PBS) for three times, and serum-free DMEM containing penicillin and streptomycin was added. Culture supernatant after 48 hours from culturing start (herein below, it is sometimes referred to as "CM") was collected to be centrifuged at 1,500 rpm for 5 minute. After that, it was centrifuged at 3,000 rpm for 3 minutes to remove other cells. Five mL of CM is mixed with 45 mL of 100 % ethanol, and the mixture was incubated for 1 hour at - 20 °C. The mixture was centrifuged at 15,000 rpm for 15 minutes at 4 °C to remove the supernatant. Precipitate from CM was resuspended in 90 % cold ethanol (- 20 °C), and centrifuged at 4 °C for 15 minutes at 15,000 rpm. Final precipitate was frozen at -80 °C to be lyophilized, and it was stored at -30 °C until the use. The serum-free DMEM which was not contacted with the cell was used as a control.

(1-4) Preparation of the Ti implant screw with immobilizes CM

Ti implant screw (5 mm of a full, 2 mm of a thread diameter, and 1 mm of pitch) was provided from Nishijima Medical Inc. The Ti implant was manufactured according to the conditions obtained from prior study.

The surfaces of Ti implants were treated with sole PBS, sole DMEM or CM, and classified into the PBS-treatment group (negative control group), the DMEM-treatment group (positive control group), and the CM/DMEM-treatment group (test group) respectively.

Each of the groups was prepared as follows.

At first, Ti implant was washed with acetone and 70% ethanol and immersed in the first calcification solution [ 50 mM of Tris-HCl (pH 7.4) containing 136.8 mM of NaCl, 3.1 mM of $CaCl_2$ and 1.86 mM of $K_2HPO_4$ (Wako Pure Chemical Industries Ltd.) at 37 °C for 4 hours.

In parallel with the immersion, three types of the second calcification containing 142.8 mM of $KNO_3$, 1.5 mM of $Ca(NO_3)_2{\cdot}4H_2O$ and 0.9 mM of $K_2HPO_4$ (Wako Pure Chemical Industries Ltd.) and any one of medium selected the group consisting of 1 mg/ml of CM/DMEM, 3 mL of DMEM, and 3 mL of PBS.

Five washed Ti implant materials were immersed in 3 mL of each type of the three calcification solutions at 37 °C for 3 days to be incubated. After finishing the incubation, each of Ti implant was taken out from the solution.

Next, the surface of the each of Ti implant were observed by using a scanning electron microscope (JEOL Ltd.) to obtain micrographs of them, which were shown in Fig 1 (A) to (F). The micrographs of Fig. 1(A) to (C) were shoot at 3,000-fold magnification and (D) to (F) at 15,000-fold magnification.

(A) to (D) showed typical topography which was appeared after the surface of Ti implant was mechanically polished. (C) and (E) showed the microstructure like snow scene. In (F), the microstructure like snow scene and tiny globule (microsphere) on the surface of Ti implant were observed. The microsphere was indicated by an arrow. The line segments in the (A) to (C) showed 10 μm of length, and those in the (D) to (F) showed 2 μm of length.

(2) Cell adherent analysis

Ti disk (10 x 10 mm; Ofa Co. Ltd.) was treated with any one of PBS, DMEM or CM by using the similar method employed for immobilization on the Ti implant. The rat BMSCs (1.0 x $10^6$ cells) were plated on the Ti disc which was treated s mentioned above (N = 3); then they were incubated in DMEM including 10 % FBS and 1 % of penicillin-streptomycin at 37 °C for 24 hours under the presence of 5 % $CO_2$.

Adhered rat BMSCs were incubated with 0.05 % trypsin - EDTA (Sigma-Aldrich) for 10 minutes to be released from Ti disc. The disc was observed by using SEM to confirm that all of the BMSCs were released. The released cells were counted by using the counting chamber (Sunlead Glass Corp.). The experiments were repeated triple times, and their statistical difference were analyzed by using one-way analysis of variance (ANOVA) in Scheffe test under the significant level of 5% ($P < 0.05$). Results were shown in Fig. 2.

Significant differences were respectively confirmed between the PBS-treatment group and the DMEM-treatment group, and between the PBS-treatment group and the CM treatment group (*: $P < 0.05$, ANOVA test).

(3) Observation of the Ti implant surface by using the scanning electron microscope (SEM)

From the above, Ti implant of which surface was treated with PBS showed the typical topography of the mechanical polishing as shown Fig. 1(A) and (D). Ti implant, on which DMEM was immobilized, displayed the microstructure like snow scene broadly and randomly (Fig. 1(B) and (E)). The surface of Ti implant coated with CM displayed the microspheres on the microstructure like snow scene (see the arrow of Fig. 1(C) and (F)). These results suggested that the topographies of the implant surface were changed depending on the immobilized components in CM or DMEM.

(4) Identification of a protein on the Ti implant surface by using LC/MS/MS

The proteins were released from the Ti implant surface of the CM/DMEM-treated group obtained as described above by using 80% acetonitrile. The released proteins were lyophilized to become powder. One mg of the protein powder was added into 100 μL of sample buffer for electrophoresis to be diluted; 10 μL of the diluted sample buffer was subjected to gel electrophoresis using 12% of acrylamide gel. After the electrophoresis, the gel was stained with CBB (Coomassie Brilliant Blue) solution or Silver Stain Kit (Thermo Inc.) respectively. After staining, the gel was sliced by using a surgical knife.

The gels sliced as mentioned above was firstly washed in 25 mM of $NH_4HCO_3$ containing 100 μL of 30% (v/v) acetonitrile for 20 minutes twice to be destained; then they were dehydrated in CVE-3100 (Tokyo Rikakikai Co., Ltd.). For in-gel digestion of protein, the gels were swollen by using 100 μL of 50 mM of iodoacetamide (Wako Pure Chemical Industries Inc.) / 100 mM $NH_4HCO_3$ (pH 7.8) containing 20 μg/mL of sequence grade trypsin (Promega KK.) for 1 hour at 37 °C; then 100 μL of 25 mM of $NH_4HCO_3$ was added to it to be incubated at 37 °C overnight. Peptide was extracted in 60 % acetonitrile containing 0.1 % TFA at room temperature for 20 minutes, and the peptide sample obtained was dehydrated in the CVE-3100.

Peptide mass fingerprinting (PMF) was performed by using Paradigm MS4-LCQ Advantage (AMR Inc.). The obtained peptide mass spectrum was utilized to generate a peptide mass list for query to Swiss-Plot protein knowledgebase (limited to mammary protein) of the Mascot search engine (www.matrixscience.com).

Search parameters were set to allow the tolerance of 1 missed trypsin cleavage, the mass tolerance of 6,100 ppm and the methionine residue oxidation. When the query matched not less than 4 peptides contained, and the statistically significant Mowse (molecular weight search) score which is generated by Mascot search engine, the identified protein was authorized.

Next, the proteins immobilized on the Ti implant and in culture medium (CM) were detected with LC/MS/MS. About 2,000 proteins were detected in the culture supernatant, and the number was mostly as the same as that on the surface of the Ti implant. All of the proteins detected on the surface of the Ti implant were also detected in CM. Since extracellular matrix contains proteins, signal transduction, protein synthesis, processing and growth factor proteins, the protein was detected by using the forward primer and reverse primer shown in below.

(Primers for amplification of ALP gene)

Forward primer: GCTGTGAAGGGCTTCTTGTC (Sequence No. 1)
Reverse primer: CGCCTATCAGCTAATGCACA (Sequence No. 2)

(The primers for amplification of OCN gene)

Forward primer: TGAGGACCCTCTCTCTGCTC (Sequence No. 3)
Reverse primer: GAGCTCACACACCTCCCTGT (Sequence No. 4)

(The primers for amplification of OPN gene)

Forward primer: AGGTCCTCATCTGTGGCATC (Sequence No. 5)
Reverse primer: AGACTGGCAGTGGTTTGCTT (Sequence No. 6)

(The primers for amplification of BSP gene)

Forward primer: TTCTCGAGAAAAATTTCCA (Sequence No. 7)
Reverse primer: TCACTGGTGGTAGTAATAAT (Sequence No. 8)

(The primers for amplification of Col-1 gene)

Forward primer: CGATGCCATTTTCTCCCTTA (Sequence No. 9)
Reverse primer: CTCTGGTACCGCTGGAGAAG (Sequence No. 10)

(The primers for amplification of GAPDH gene)

Forward primer: ATGACTCTACCCACGGCAAG (Sequence No. 11)

Reverse primer: TTCAGCTCTGGGATGACCTT (Sequence No. 12)

[Table 1]

| gene | size(bp) | Seq. No. of Forward primer | Seq. No. of Reverse primer | Accession No. |
|---|---|---|---|---|
| ALP | 624 | 1 | 2 | NM_013059 |
| OCN | 151 | 3 | 4 | M25490 |
| OPN | 518 | 5 | 6 | XM_002728077 |
| BSP | 916 | 7 | 8 | L06562 |
| Col-I | 578 | 9 | 10 | NM_053356 |
| GAPDH | 529 | 11 | 12 | AF106860 |
| ALP: alkaline phosphatase, OCN: osteocalcin,<br>OPN: osteopontin, BSP: bone sialoprotein, Col-I: type I collagen | | | | |

Type I collagen, bone I collagen, bone sialoprotein 2, decorin, oseteopontin, oseteocalsin, fibronectine, which were as bone- related proteins and vascular endothelial growth factor A (VEGF-A) were detected. The results were shown in Fig. 2.

[Table 2]

| Protein name | Protein database accession No. | pI / molecular weight (Da) | Location | Seq. No. |
|---|---|---|---|---|
| collagen α-1(I) chain precursor | NP_445756 | 5.71/137953.45 | 10q31 | 13 |
| collagen α-1(II) chain precursor | NP_037061 | 8.46/134570.01 | 7q36 | 14 |
| collagen α-1 (XXVII) chain precursor | NP_942042 | 9.73/187811.56 | 5q24 | 15 |
| collagen α-2(I) chain precursor | NP_445808 | 9.39/129564.06 | 4q13 | 16 |
| fibronectin | NP_062016 | 5.44/272446.09 | 9q33 | 17 & 18 |
| decorin | NP_077043 | 8.96/39805.10 | 7q13 | 19 & 20 |
| osteocalcin | NP_036994 | 9.27/12036.68 | 4q43 | 21 |
| osteopontin | NP_037013 | 4.35/34962.99 | 14p22 | 22 |
| bone sialoprotein 2 | NP_036719.2 | 6.26/2038.25 | 14p22 | 23 |
| VEGF-A | NP_114024.2 | 4.00/2805.19 | 6q31 | 24 |

(5) RNA analysis of rat BMSCs in vitro

The rat BMSCs were incubated on the Ti disc treated with any one of PBS, DMEM or CM. The immobilized method and culture method were the same as those used in the cell adhesion analysis. The rat BMSCs were cultured for 1 day, 7 days or 14 days. Total RNA of the rat BMSCs cultured on each of the disc were assayed by using TRIZOL reagent (Invitrogen Life Technologies Inc.) according to the protocol of the manufacturer. cDNA was synthesized from 1 μg of the total RNA in the reaction mixture containing 10 x reaction buffer, 5 mM of dNTP mixture, 1 U/μL of RNase inhibitor, 0.25 U/μL of reverse transcriptase (M-MLV reverse transcriptase, Invitrogen Life Technologies Inc.) and 0.125 μM of random primer (Takara Bio Inc.).

For sq-PCR, the assay was performed under the condition of 95 °C for 30 seconds, 45 to 60 °C for 30 seconds, and 72 °C for 30 seconds, and the cycle was repeated for 25 to 35 cycles in PCR Thermal Cycler SP (Takara Bio Inc.).

Rat Glyceraldehyde-3-phosphate dehydrogenase (GAPDH) primer was used as an internal standard. The expression level of mRNA to GAPDH in the transplanted culture cells (%) was measured by using Scion Image picture-imaging software (Scion Corporation). The synthesized cDNA was used as the template for subsequent PCR amplification, when the specific primers shown in Fig. 1 were used. All of the experiments were repeated triplicate, and the data was analyzed by using the one-way analysis of variance (ANOVA) with Scheffe test at the significant level of 5%.

The results were shown in Fig. 3 as the gene expression pattern of OCN, OPN and Col 1 (see Fig. 3 (A) to (C)), and quantified graph of them (see Fig. 3(D) to (F)).

(6) CM labeling by using Qdot (registered trade mark) 655-ITK carboxyl quantum dots (QDs)

CM labeling by using QDs (Invitrogen Molecular Probes Inc.) was performed according to the instruction manual of the manufacture. Briefly speaking, Ti sample treated with the calcification solution was added into the mixture comprising 25 μL of 8 μM of QDs, 1 mg/mL of CM, 1 mL of 10 mM of borate buffer (pH 7.4) and 5.7 μL of 10 mg/mL of N-ethyl-N'-dimethylaminopropyl-carbodiimide (a cross-linking agent: Sigma- Aldrich Co. LLC.) for gently stirring for 1 hour at room temperature. QD labelings of the serum free DMEM or PBS were used as controls. The results were shown in Fig. 4.

In the figure, A, B and C show the image of Ti implant before test respectively. A1 shows the image of 1 day, A7 shows 7 days, A14 shows 14 days, and A28 shows 28 days after the start. B and C show the images at the same date.

In the PBS-treated Ti implant, any fluorescence was not detected around it through the observation period. In contrast, in the DMEM-treated or the CM-treated Ti implant, the fluorescence was detected around it.

Accordingly, it was found out that a localization of biomolecule was occurred on the DMEM-treated or the CM-treated Ti implant.

**(EXAMPLE 2)** A preparation of the virus for producing the immortalized dens deciduous stem cell

(1) The reagents for the plasmid extraction

(1-1) The reagents and the like

Kanamycin (Kan), ampicillin (Amp), LB liquid medium and LB agar medium, glycogen, agarose, sterilized water, ammonium acetate, sodium acetate, dodecyl sodium sulfate and RNase A were used. Both of kanamycin (Kan) and ampicillin (Amp) with 50 mg/mL were prepared and stored at -20 °C as stock solution. Glycogen was prepared as 20 mg/mL solution. 10 mg/mL of RNase A was prepared and stored at -20 °C. 10 M of saturated ammonium acetate ($NH_4OAc$) and 3M of sodium acetate (NaOAc; pH 5.2) were prepared.

(1-2) Restriction enzymes and the like

E. coli competent cell (Supercharge EZ10 Electrocompetent Cells, the product code 636756), Swa-I (the product code 1111A, Smi-I is equivalent enzyme), Xho-I (the product code 1094A), T4 DNA Ligase (the product code 2011A), NucleoBond Xtra Midi (the product code 740410.10/.50/.100), and NucleoSpin Plasmid (the product code 740588 10/50/250) were purchased from Takara Bio Inc. Pac I was purchased from New England Biolabs Japan Inc.

(1-3) Buffers and the like

1 x TE Buffer (10 mM Tris-HCl [pH 8.0] containing 1 mM of EDTA), the mixture comprising of phenol, chloroform and isoamyl alcohol (ratio is 25:24:1, herein below, it is referred as "PCI mixture"), which was saturated with 100 mM Tris-HCl (pH 8.0), was prepared. Ethanol was used at the concentration of 100 % or 70%. In order to purify pAdeno-X plasmid DNA used for mini-scale recombination, the following buffers 1 to 4 were prepared.

Buffer 1: 25 mM of Tris-HCl (pH 8.0) containing 10 mM of EDTA and of 50 mM glucose (after autoclave, stored at 4 °C)
Buffer 2: 0.2 M of NaOH containing 1 % SDS (prepared at the time of use, stored at room temperature with seal)
Buffer 3: 5 M KOAc (after autoclave, stored at 4 °C)
Buffer 4: 10 mM of Tris-HCl (pH 8.0) containing 1 mM of EDTA and 20 μg/mL of RNase (RNase was added at the time of use, stored at -20 °C)

(2) The reagents for the adenovirus purification and β-gal assay

Human adenovirus V transformed HEK293 cell (ATCC #CRL 1573) was used. HEK293 was incubated in a complete medium. The composition of the complete medium was DMEM containing 100 unit/mL of sodium penicillin G, 100 μg/mL of streptomycin, and 4 mM of L-glutamine, supplemented with 10% FBS. Sodium penicillin G solution was prepared at 10,000 unit/mL and streptomycin sulfate solution was prepared at 10,000 μg/mL, and they were stored as stock solutions.

For culturing, a plate having 60 mm ϕ, that having 100 mm ϕ, 6-well plate, T75 flask and T175 flask were used.

Trypsin-EDTA (the product code: CC-5012) was purchased from Takara Bio Inc. Phosphate buffered saline (PBS: without $Ca^{2+}$ and $Mg^{2+}$) and Dulbecco's phosphate buffered saline (DPBS, with $Ca^{2+}$ and $Mg^{2+}$) were prepared. Also, 0.33 % neutral red stain solution, and 0.4 % trypan blue stain solution were used.

In β-gal assay, X-Gal (5-bromo-4-chloro-3-indolyl-β-D-galactopyranoside (25 mg/ml)) in dimethylformamide (DMF) solution was stored at -20 °C in a shading state. Luminescent β-gal Detection Kit II (the product code 631712, Takara Bio Inc.) was used.

(3) Preliminary test

(3-1) Construction of recombinant adenovirus including lacZ (pAdeno-X-lacZ)

After thawing and deleting DMSO, HEK293 cells were resuspended in 10 mL of the complete medium, and then whole amount of the medium was transferred into the culture plate having the diameter of 100 mm. After HEK293 cells adhered to the plate, the culture medium was removed. Then, the cells were washed once with sterile PBS, and then treated with 1 ml of trypsin-EDTA about 2 minutes.

Next, 10 ml of the complete medium was added to stop trypsinization, and then gently suspended. By conducting viable count, $10^5$ cells were transferred into the plate having 100 mm diameter including 10 mL medium to spread out evenly.

pShuttle2-lacZ (a positive control vector included in Adeno-X Expression System 1) and Adeno-X Viral DNA (both PI-Sce I and I-Ceu I digested) included in the kit was used. According to the protocol attached in the kit, the recombinant adenovirus including lacZ was constructed. The target cell, SHED, was infected with the construct to assay the expression of β-garactosidase to confirm that the vector was constructed.

(3-2) Construction of the recombinant pShuttle2 plasmid

Prior to the construction of the recombinant pShuttle2 Vector (herein below, it is referred to as "rpShuttle2 Vector".); E. coli DH5α was transformed with pShuttle2 Vector and pShuttle2-lacZ Vector, included in the kit. Transformants were selected on LB agar plate including 50 μg/ml kanamycin (herein below, it is referred to as "LB/Kan".). Bacterial cells obtained from a single colony were streaked on a new LB/Kan to be incubated at 37 °C for overnight.

Next, hTERT, bmi-1, E6, and E7 were cloned into pShuttle2 according to the following procedure. pShuttle2 vector was cleaved by using a restriction enzyme suitable for these genes.

Next, referring to pShuttle2 Vector Information Packet (PT3416-5) attached to the kit, multi cloning site matching DNA to be inserted was decided. The plasmid treated with the restriction enzyme was treated by using alkaline phosphatase to be purified.

According to the conventional method, target DNA fragments were prepared to be purified. The vector digested with the restriction enzyme and the gene fragments were ligated. By using the ligation product, DH5α cells (the competent cell) were transformed. A portion of the competent cell was taken to be transformed by using a control vector, pShuttle2-lacZ Vector included in the kit, to use as the positive control.

The mixture including transformed E. coli was plated on the LB/Kan agar plate to select kanamycin resistant (Kanr) transformant (a colony). From 5 to 10 Kan resistant clones were selected to be plated in a small amount of the liquid medium to be amplified. After confirmation that these clones have rpShuttle2 Vector, they were incubated overnight. Then, by using a commercially available silica gel adsorption column, the constructed plasmid DNA was purified according to the conventional method.

The plasmid DNA was treated with the restriction enzyme to be subjected to 1 % agarose gel electrophoresis; thereby the target recombinant plasmid was identified. By sequencing, the direction of the inserted fragment and inserted site were confirmed to identify the positive clone.

The recombinant pShuttle2 plasmid DNA (herein below, it is referred to as "rpShuttle2 plasmid DNA".) was directly transfected into the target cell, and then it was subjected to western blot to check the target protein expression preliminary.

(3-3) Double digestion of rpShuttle2 plasmid DNA with PI-Sce I/I-Ceu I

From the rpShuttle2 plasmid DNA produced as mentioned above, an expression cassette of the inserted gene was taken out by using PI-Sce I and I-Ceu I. According to in vitro ligation method written in the protocol attached to the kit, the expression cassette which was taken out was integrated into Adeno-X Viral DNA. PI-Sce I/I-Ceu I double-digestion solution for the rpShuttle2 plasmid DNA was prepared 30 μL. The reagents shown in the following table 1 was entered into 1.5 ml of the sterilized micro centrifuge tube to be mixed.

[Table 3]

| Reagent and others | Tube 1 | Tube 2 (lacZ control) |
|---|---|---|
| Sterilized water | 19.5 μl | 19.5 μl |
| 10 x double-digention solution | 3.0 μl | 3.0 μl |
| rpShuttle2 plasmid DNA (500 ng/μl) | 2.0 μl | - |
| pShuttle2-lac Z plasmid (500 ng/μl) | - | 2.0 μl |
| PI-Sce I (1 unit/μl) | 2.0 μl | 2.0 μl |
| I-Ceu I (5 unit/μl) | 0.5 μl | 0.5 μl |
| 10 x BSA | 3.0 μl | 3.0 μl |
| Total | 30.0 | 30.0 |

Next, after fully mixing, the micro centrifuge tube was lightly centrifuged, and then incubated for 3 hours at 37 °C. The double digested reaction mixture (5 μL) was subjected to 1 % agarose/EtBr gel electrophoresis together with 1 kb ladder (a DNA size marker).

(3-4) Extraction by phenol: chloroform: isoamyl alcohol

The remains of the double-digestion solution (25 μL), 70 μL 1 x TE Buffer (pH 8.0) and 100 μL PCI mixture were added into the centrifuge tube, and mixed by using a vortex. Then, the tube was centrifuged by using a micro centrifuge at 4 °C with 14,000 rpm for 5 minutes. Then, the aqueous layer was transferred to 1.5 ml of a clean centrifuge tube, to which 400 μL of 95 % ethanol, 25 μL of 10 M ammonium acetate, and 1 μL glycogen (20 mg/ml) were added, and then fully mixed by using the vortex.

Next, the tube was centrifuged at 4 °C with 14,000 rpm for 5 minutes. Then, the supernatant was deleted by aspiration to obtain a pellet. 300 μL of 70 % ethanol was added on the pellet, it was centrifuged for 2 minutes with 14,000 rpm. The supernatant was carefully aspirated to remove, the pellet was air dried about for 15 minutes. After the pellet was dried, it was dissolved in 10 μL of sterilized 1 x TE Buffer (pH 8.0), and the solution was stored at -20 °C until use.

(4) Construction of the recombinant Adeno-X plasmid DNA (4-1) Subcloning of the expression cassette into Adeno-X virus genome

The reagents shown in the following table 4 were sequentially added into the 1.5 ml of the sterilized micro centrifuge tube. Then, it was mildly mixed and lightly centrifuged. After that, it was incubated at 16 °C for overnight.

[Table 4]

| Reagent and others | volume (μL) |
|---|---|
| PI-Sce I/I-Ceu I digested pShuttle2 plasmid DNA | 2.0 |

(continued)

| Reagent and others | volume (μL) |
|---|---|
| PI-Sce I/I-Ceu I digested pShuttle2-lac Z plasmid DNA | - |
| sterilized water | 3.0 |
| 10 x DNA Ligation Buffer | 1.0 |
| Adeno-X Viral DNA(250 ng/μl) | 3.0 |
| DNA Ligase(1 unit/μL) | 1.0 |
| Total | 10.0 |

To each sample, 90 μL of 1 x TE Buffer (pH 8.0) and 100 μL of PCI mixture were added, and then they were mildly mixed by using vortex. They were centrifuged at 4 °C with 14,000 rpm for 5 minutes. The aqueous layers were transferred to 1.5 mL of the clean micro tubes, to which 400 μL of 95 % ethanol, 25 μL of 10 M of ammonium acetate solution, and 1 μL of glycogen (20 mg/ml) were added, and then they were mildly mixed by using the vortex.
They were subjected to the centrifugation at 4 °C for 5 minutes with 14,000 rpm, and the supernatant was removed by the aspiration to obtain the pellet. The following ethanol precipitation operations were the same as those of (3-4).
After drying the pellet, it was dissolved in 15 μL of the sterile deionized water.

(4-2) Swa I digestion of the recombinant Adeno-X plasmid DNA

The digestion solution as shown in the following table 5 was prepared, and added into each sample in the centrifuge tube. Then, they were incubated for 2 hours at 25 °C.

[Table 5]

| Reagent and others | Volume (μL) |
|---|---|
| ligation product | 15 |
| 10 x Swa I Digestion Buffer | 2.0 |
| 10 x BSA | 2.0 |
| Swa I (10 units/μL) | 1.0 |
| Total | 20.0 |

To each sample, 80 μL of 1 x TE Buffer (pH 8.0) and 100 μL of PCI mixture were added, and then it was mildly mixed by using the vortex. It was centrifuged at 4 °C with 14,000 rpm for 5 minutes. The following ethanol precipitation operations were the same as those of (3-4), and the dissolved solution of the pellet was stored at -20 °C until use.

(4-3) Confirmation of the E. coli transformant by the recombinant Adeno-X plasmid DNA

The electroporation competent E. coli was transformed with the Swa I digested products obtained in (4-2) by using Supercharge EZ10 Electrocompetent Cell (the product code 636756).
The transformant mixture was plated on the agar plate, which is the mixture of LB medium and ampicillin (final conc. 100 μg/mL) (herein below, it is referred to as "LB/Amp agar plate".), and then incubated at 37 °C for overnight to select ampicillin resistant (Ampr) transformant. About $10^6$ of colonies were obtained. The obtained colonies were checked by using Adeno-X System PCR Screening Primer Set attached to the product.
The bacterial cells from the single colony were plated in 5 mL of fresh LB/Amp liquid medium, and incubated overnight. The next day, according to the mini-scale method as mentioned below, Adeno-X plasmid DNA was purified.

(4-4) Mini-scale preparation of the recombinant Adeno-X plasmid DNA

5 mL of log-phase culture medium was centrifuged with 14,000 rpm for 30 seconds to remove the supernatant. The pellet was centrifuged again at 10,000 rpm for 1 minute, and then the supernatant was removed by using the micropipette.

150 $\mu$L of the buffer 1 was added to the pellet in the tube, and mildly pipetted to resuspend. 150 $\mu$L of the buffer 2 was added into the cell suspension. Then the cell suspension were mildly inverted to mix and stood for 5 minutes on ice. 150 $\mu$L of the buffer 3 was added to the cooled cell suspension, and then it was inverted to mix again and stood for 5 minutes on ice.

The cell suspension was centrifuged at 4 °C with 14,000 rpm for 5 minutes, and the clear supernatant was transferred into 1.5 ml of the clean centrifuge tube. 450 $\mu$L of PCI mixture was added to the supernatant, and then inverted to mix. Then, it was centrifuged at 4 °C with 14,000 rpm for 5 minutes, and the aqueous layer was transferred to the clean 1.5 ml of the micro centrifuge tube.

The following ethanol precipitation operations were the same as those of (4-1), and the dissolved solution of the pellet was stored at -20 °C until use. The rDNA of the interest was identified by using the analysis with the restriction enzymes and PCR as described below.

(5) Restriction site analysis of the obtained rAdeno-X plasmid DNA

Analysis was performed by using PI-Sce I and I-Ceu I. The reagents shown in the following table 6 was entered into 1.5 ml of the micro centrifuge tube. Then, 30 $\mu$L of PI-Sce I/I-Ceu I double digestion solution was added to it, and then fully mixed and then it was lightly rotated to collect the contents.

[Table 6]

| Reagent and others | Volume ($\mu$L) |
|---|---|
| sterilized water | 19.5 |
| 10 x double-digention solution | 3.0 |
| rpAdeno-X DNA (500 ng/$\mu$l)(500 ng/$\mu$l) | 2.0 |
| pShuttle2-lac Z plasmid (500 ng/$\mu$l) | - |
| PI-Sce I (1 unit/$\mu$l) | 2.0 |
| I-Ceu I (5 unit/$\mu$l) | 0.5 |
| 10xBSA | 3.0 |
| total | 30.0 |

It was incubated at 37 °C for 3 hours to perform restriction treatment. The reaction mixture after the treatment was subjected to 1% agarose /EtBr gel electrophoresis.

(6) Production of the recombinant adenovirus

(6-1) Preparation of the rAdeno-X plasmid DNA for HEK293 cell transfection

The reagents shown in the following table 7 was entered into the 1.5 ml of the sterilized centrifuge tube to be mixed, and then it was lightly centrifuged by using the micro centrifuge. Then, it was incubated at 37 °C for 2 hours to treat the rAdeno-X plasmid DNA with Pac I restriction enzyme.

[Table 7]

| Reagent and others | Volume ($\mu$L) |
|---|---|
| Sterilized water | 20 |
| pAdeno-X plasmid DNA (500 ng/$\mu$l) | 10 |
| 10 x Pac I Digestion Buffer | 4 |
| 10 x BSA | 4 |
| Pac I (10 units/$\mu$L) | 2 |
| total | 40 |

60 $\mu$L of 1 x TE Buffer (pH 8.0) and 100 $\mu$L of PCI mixture were added to it, and then they were mildly mixed by using the vortex. Then, it was centrifuged by using the micro centrifuge at 4 °C for 5 minutes with 14,000 rpm. The aqueous layer was carefully transferred into 1.5 ml of the clean sterilized centrifuge tube.

The following ethanol precipitation operations were conducted as the same as those of (3-4), and the dissolved solution of the pellet was stored at - 20 °C until use.

(6-2) Transfection of Pac I digested Adeno-X plasmid DNA into HEK293 cell

Before 24 hours of the plasmid DNA transection, HEK 293 cells were plated on the culture plate having the 60 mm diameter so as that the cell number was about 1 to 2 x $10^6$ (about 100 cells/$mm^2$). Then, they were incubated at 37 °C under the presence of 5% $CO_2$.

10 μL of Pac I-digested Adeno-X plasmid DNA was transfected to each culture plate to introduce Adeno-X DNA into the HEK293 cell, according to a standard transfection method (CalPhos Mammalian Transfection Kit, the product code 631312, Takara Bio Inc.). Occurrence of CPE (cytopathic effect) was confirmed from the next day of the transfection.

One week later, the cells adhered on the bottom or side wall of the culture plate was released by mild mixing. The obtained cell suspension was transferred into 15 mL of the sterilized centrifuge tube having a conical bottom, and it was centrifuged at room temperature for 5 minutes with 1,500 x g.

Obtained precipitate was suspended in 500 μL of the sterilized PBS. The solution was subjected to the free-thaw operation for 3 times, which is frozen in dry ice/ethanol and thawed in the incubator with 37 °C, to obtain the lysate in which the cells were fully thawed. Next, it was lightly centrifuged to remove suspended matter, and then the supernatant was transferred into the sterilized another tube to use immediately. The lysate did not use immediately was stored at -20 °C.

250 μL of the lysate was added onto the cultured cells in the plate with 60 mm diameter, and continued to culture. Note that by using anti-Hexon antibody included in Adeno-X Rapid Titer Kit (the product code 631028, Takara Bio Inc.), the adenovirus was titrated according to the instruction manual (PT3651-1) of the kit.

(6-3) Virus amplification for preparing the virus having high titer

Before 24 hours of the titration start, HEK293 cells were plated on a T75 flask, and they were incubated at 37 °C in the presence of 5% $CO_2$ for overnight to be confirmed that they became 50 to 70% of confluent.

In the next day, the medium was exchanged to fresh one including the virus for infection with the virus at MOI = 10. After the incubation at 37 °C in the presence of 5% $CO_2$ for 90 minutes, the flask was taken out and 10 mL of the medium was added into the flask.

They were cultured at 37 °C for 3 to 4 hours in the presence of 5% $CO_2$, and CPE was confirmed. After released 50% of the cells, the released cell suspension was prepared as described above; it was transferred to 15 mL of the sterilized centrifuged tube with the conical bottom. The freeze and thaw operation as described above was performed, and the cells were thawed. By using Adeno-X Rapid Titer Kit (the product code 631028), the titer, $10^7$ PFU/mL was obtained.

Western blotting was performed, and it was confirmed whether the packaged adenovirus genome had copies of the specific transcription unit against the target gene as the functional form or not.

(7) Adenovirus infection to the target cells

(7-1) Infection to the target cells

Before 24 hours of the infection, 1 x $10^6$ cells of SHED were plated on 6-well plate. In the next day of the plating, the medium was removed, and 1.0 mL of the medium including virus was added to the center of each well in the plate. The solution was spread on a monolayer formed by the SHED.

It was incubated at 37 °C for 4 hours under the presence of 5% $CO_2$, and the virus was infected to SHED. Next, the fresh medium was added, and then incubated at 37 °C in the presence of 5% $CO_2$. From 24 to 48 hours after the infection, the expression of the introduced gene was analyzed time dependently.

(7-2) Analysis of the β-galactosidase expression of the infected cells

The β-galactosidase expression in the adherent cell infected with the Adeno-X-lacZ was assayed by using Luminescent β-gal Detection Kit II (the product code 631712, Clontec Laboratories Inc.).

(Example 3) Manufacturing of SHED

(1) Preparation of exfoliated dens deciduous cell

An exfoliated dens deciduous from 10 years old healthy boy was used. After the exfoliated dens deciduous was disinfected with Isodine solution, a crown of the teeth was horizontally cut by using the dental diamond point, and then the dental pulp tissue was collected by using the dental reamer. The obtained dental pulp tissue was digested in the solution including 3 mg/mL of type I collagenase and 4 mg/mL of disperse at 37 °C for 1 hour. Next, the solution was filtrated by using 70 mm of cell strainer (Falcon Inc.).

The filtrated cells were resuspended in 4 mL of the medium to be plated into the culture dish having the 6 cm of diameter. DMEM including 10 % FCS was added into the dish and then cultured for about 2 weeks in the incubator adjusted to 5% $CO_2$, at 37 °C. The adherent cells formed colonies (the dental pulp stem cells) were treated by using 0.05% trypsin-EDTA for 5 minutes at 37 °C, and then the cells released from the dish were collected.

Next, the adherent cells selected as mentioned above were plated on the culture dish for the adherent cells (a collagen coat dish), and they were incubated in the incubator prepared under 5% $CO_2$ at 37 °C as the primary culture to obtain the primary cultured cell. When the cells became macroscopically sub-confluent (about 70 % of the surface of the culture container was covered by the cells), or confluent, the cells were treated by using 0.05% trypsin-EDTA at 37 °C for 5 minutes to be released from the container, and then collected.

Thus obtained cells were again plated on the dish including the medium, and performed passage in several times to be grown to achieve the cell number, about 1 x $10^7$ cells/mL. The obtained cells were stored in the liquid nitrogen.

After that, by using the primary cultured cells, the passage was performed at the cell concentration at 1 x $10^4$ cells/$cm^2$. In the experiment, the cells passed from 1 to 3 were used. The human BMMSC (the bone marrow mesenchymal stem cell, Bone Marrow Mesenchymal stem cells) was purchased from Lonza Group Ltd. and cultured according to the instruction manual provided from the manufacturer.

As described above, the human exfoliated dens deciduous dental pulp stem cells (SHED) were obtained. Among the obtained SHED, about 1 x $10^6$ cells of STRO-1 expression cells were sorted from each sample by using FACSTARPLUS (Becton, Dickinson and Company).

According to the manufacturer's instruction manual of the bromodeoxyuridine BrdU staining kit (Invitrogen Inc. ), BrdU was incorporated into the cells during 12 hours to evaluate the growth rate of SHED (n = 3 in each group). The experiments were repeated for 5 times. After one-way analysis of variance, Tukey-Kramer test was performed to evaluate statistical significant difference.

In order to detect STRO-1 with immunofluorescence, SHED was fixed by using 3% paraformaldehyde, and rinsed twice with PBS and then treated by using 100 mM of glycine for 20 minutes. Next, these cells were permeabilized with 0.2% of Triton-X (Sigma-Aldrich Co. LLC.) for 30 minutes. Then, they were incubated in the mixture of 5 % donkey serum and 5% of bovine serum albumin for 20 minutes.

Next, the cells were incubated with the primary antibody, mouse antihuman STRO-1 antibody (1:100, R&D Inc.) for 1 hour, then incubated with the secondary antibody, goat anti-mouse immunoglobulin M-FITC antibody (1:500, Southern Biotech Corp.) for 30 minutes, and then mounted by using Vector Shield DAPI (Vector Laboratories Inc.).

After that, $\alpha$-MEM supplemented with 15 % of FBS was added to the 6 well plate, and then the sorted cells were plated in each well for preparing clones. About 300 colonies among the proliferated cells were pooled for the test.

(2) Transgenesis

As described above, 4 genes, bmi-1, E6, E7 and hTERT were integrated into the adenovirus vector to manufacture a virus vector to express the gene products. As a reference, the control vector to which these genes were not integrated was manufactured.

SHED was plated on the collagen coat dish having 100 mm $\phi$ at the concentration of 1 x $10^6$ cells, and then DMEM supplemented with 10% FBS was added. They were cultured until sub-confluent. The medium was removed by aspiration, and 500 $\mu$L of the virus solution diluted with the medium was added (MOI = 10), and then incubated at 37 °C for 1 hour in the 5% $CO_2$ incubator for the virus vector infection. After 48 hours from the infection, the medium was exchanged to the medium supplemented with puromycin (1 pg/mL), and the infected cells were incubated for 10 days in the medium to select the resistant clone. The resistant clones, 500 to 600, were pooled. In every 3 to 4 days, about 0.5 x $10^5$ cells of SHED was plated to the culture dish having 100 mm$\phi$ to perform passage. SHED to which the genes were transferred was named SHED-T, and that to which the genes were not transferred was named SHED-C.

(3) Measurement of the growth rates of SHED-C and SHED-T

Status of the population doubling time of SHED-T (the gene transferred SHED) was shown in Fig. 5. In the figure, a vertical axis shows the population doubling time number (cell division number, times), and an abscissa axis shows the time period (date of culture). Evaluation standard for the aging was the status wherein SHED in culture does not divide for 1 month.

The growth of SHED-C has stopped about 30 times to enter aging or proliferation termination phase. In contrast, SHED-T passed over and proliferated after 800 days has passed

(3-1) Flow cytometry analysis

In order to obtain a single cell suspension, the adherent monolayer cells were digested with trypsin/EDTA. The anti-STRO-1 monoclonal antibody (1:100) was added to 2 x $10^5$ cells and stood to analyze by using FACS Calibur flow cytometer (Becton, Dickinson and company). When the fluorescence level of them was higher more than 99 % in the ratio compared to the control antibody with corresponding to the same isotype, the expression was positive. In both of SHED-T and SHED-C, the primary and later passage cells were fixed, and stained with FITC binding STRO-1 antibody. Then, it was analyzed by using the flow cytometry. The test was repeated twice. In SHED-C, the ratio of the STRO-1 positive cells was 27% at PD20, and decreased 15% at PD30 (Fig. 6(A) and (B)). The ratio of the STRO-1 positive cells in SHED-T was 46% at PD20 and 41% at PD40, respectively (Figs. 6(C) and (D)).

(3-2) Study for the differentiation ability

The differentiation abilities of SHED-C or SHED-T at PD0, PD10 and PD20 were studied by using the forming ability of the new bone mass and histological stain of the tissue.

Firstly, 2.0 x $10^6$ cells of SHED-C or SHED-T were mixed with 40 mg of ceramic powder of hydroxyapatite/tricalcium phosphate (HA/TCP) (Olympus Corporation), and then the mixture was inoculated subcutaneously under a dorsal surface of immunocompromised mouse at 10 weeks age (NIH-bgnu-xid, female, Harlan Sprague Dawley Inc.).

Eight weeks after the inoculation, the inoculant was recovered, and fixed by using 4% formalin to decalcify. Then, it was buffered by using PBS solution including 10% EDTA for paraffin embedding. A part of it was stored in 70% ethanol for embedding in resin.

A paraffin section was deparaffinized, and hydrated. After that, the section was stained with hematoxylin and eosin (herein below, it is referred to as "H&E".). Figs. 7(A) to (C) show the stained images of SHED-T (the immortalized stem cell) at PD0 to PD20, and Figs. (D) to (F) show the stained images of SHED-C (the normal cell) at PD0 to PD20. In order to determine the new born formation in vivo, the specified positions were chosen, and the area of the new born and the sight area were calculated to obtain the new born mass from these values for the inoculant formed after SHED-T inoculation or SHED-C inoculation.

$$\text{New born mass} = \text{New born area/sight area} \times 100$$

Fig. 8 shows the change of the new born mass of SHED-T and SHED-C at each population doubling number (doubling time). In the figure, ** shows p <0.05, *** shows p<0.01. Note that the new born mass was obtained by using the following equation.

As shown in Fig. 8, the new bone mass was decreased depending on the increase of the population doubling time in SHED-C, and it was decreased to about 1/5 at PD 20 compared to that of PD0. In contrast, the bone mass was not changed by PD 20 in SHED-T, and the bone mass in SHED-T showed 5 times higher than that of SHED-C at PD 20.

(3-3) Evaluation of canceration activity

1 x $10^6$ cells of SHED-C cells or SHED-T cells were inoculated to the subcutaneous tissue of the immune compromised mice. After inoculation, we performed the observation more than 30 days. However, the tumor was not formed during the observation term in any mice to which the cells were transplanted. Also, all of the clones from the cultured cells did not show any morphological change between the ranges from 40 to 200 PD in SHED-T cells.

By this, it was demonstrated that SHED-T had no canceration activity.

(4) Evaluation

It was demonstrated that SHED-T had proliferation ability, holding differentiation ability even after 260 PD. However, SHED-C had the differentiation ability, but had aged not over than 30PD.

As described above, it was demonstrated that SHED-T became the immortalized stem cell, and is suitable for large scale production of SHED supernatant having higher activity.

(5) Preparation of Ti implant screw fixed with CM

Preparation of Ti implant screw fixed with CM, analysis of cell adhesion, observation of the change of the surface of Ti implant with scanning electron microscopy (SEM), protein identification of the surface of Ti implant with LC/MS/MS and RNA analysis of SHED in vitro were performed as described experiment 1.

**(Example 4)**

(1) Implant and surgeon protocol

Ti implant (titanium fixture) was inserted into the femur as described (the cited reference No. 40). Rats were anesthetized with the combination of inhalation of diethyl ether mist and intraperitoneally administration of pentobarbital. Ten mm of opening was made over distal femur and the bone was bared.

Unicortical implant floor (unicortical implant floor) was formed at 7 mm distance from the distal end of the femur with a dental round bar (dental round bar, 1.5 mm of the diameter) at a rotation speed less than 1,500 rpm. The implant treated with CM, DMEM or PBS was inserted into a cortical bone to be embedded. After that, soft tissue was returned the original normal position and the opening was sutured with 3-0 VICRYL (registered trademark) SH-1 (Ethicon LLC).

(2) In vivo imaging analysis

The Ti implant having immobilized QD-modified CM was tracked and visualized with an imaging system (IVIS spectrum, XENOGEN CORP). The rat was killed at the day 1, day 7, day 14 and day 28 after implant respectively and the femur having the embedded Ti implant was taken out from each rat. QD-label associated with the taken out Ti implant in the femur was detected by the imaging.

Each of the rats and the taken out femur were imaged under the following conditions:

Height of sample: 1.50 cm
Field of view: 13 x 13 cm
Exposure time: 1 sec
f-stop: 1
Binding: 8
Excitation filter: 605 nm
Emission filter: 655 nm or, the height of sample: 0.01 cm
Field of view: 6.5 x 6.5 cm
Exposure time: 2 sec
f-stop: 1
Binding: 8
Excitation filter: 605 nm
Emission filter: 655 nm

(3) Measuring the removal torque (N cm)

The removal torque (N cm) of the femur implant was measured at the day 1, day 7, day 14 and day 28 after implant setting under deep anesthesia every experimental group (N=5 at each day). The removal torque was measured with torque gauge ATG 3 CN (registered trademark, measuring range: 0.1-3 Ncm; Tohnichi, Tokyo, Japan) and BTG 15 CN-S (registered trademark, measuring range: 2-15 Ncm; Tohnichi, Tokyo, Japan). The date was analyzed by ANOVA using Scheffe test at the significant level of 5% ($P<0.05$).

The results were shown in Fig. 9. Between the negative control group (PBS-treatment group) and the positive control group (DMEM-treatment group), the significant differences were shown both at the day 1 and the day 7; however, it was not shown at the day 14. The test group (CM-treated group) showed the higher values at the every measuring day compared with the control group, and the significant differences were shown by the day 14 (* shows $p<0.05$).

(4) Histological treatment and measurement of the bone-implant contact ratio (BIC)

After measuring the removal torque, the rats (N = 5 in each group) were killed under the deep anesthesia to take out the femur implants. The obtained sample was embedded in Technovit 7100 (Oukenshohji Co. Ltd.,). Each block was moved along the long axis direction of the implant to prepare the section having 50 μm of thickness, which was stained with toluidine blue in the conventional method.

The microscopic image of the section was displayed on the monitor to be input in the computer, and analyzed with software for image analysis (VMS-50 VideoPro, Innotech Corp.). The bone contact ratio was calculated by using the following formula:

The bone contact ratio (%) = direct implant – bone contact / periimplant length

The data was analyzed by the one-way analysis of variance with Scheffe test at the significant level of 5%. The results were shown in Fig. 10.

The result of the cancellous bone was shown in Fig 10(A) and that of the cortical bone was shown in Fig. 10(B). In the bone implant contact ratio, the cancellous bone showed the significant difference between the negative control and each of other two groups at both of the day 7 and the day 14 after implant. On the other hand, the cortical bone showed the significant difference of that between the test group and the negative control group from the day 1 after the implant (analyzed by ANOVA, * shows < 0.05, **shows < 0.01)

Since the test group showed higher BIC value, it was indicated that the unification with the bone and the implant was promoted.

**(Example 5)** Preparation of the sophisticated implant for human (1) Material and Method

Titanium implant manufactured by Astra Tech AB was used as the implant (the length 13 mm and the diameter 3.75 mm, or the length 11 mm and the diameter 3.75 mm).

(2) Preparation of the culture supernatant containing the growth factors

The solution containing the growth factors was prepared by using the culture supernatant of the stem cell obtained in the example 1 and treated as follows.

The culture supernatant was put into the culture flask, and then the titanium implant material was added. The culture flask was sealed tightly to be shaken fully. The flask was sonicated with a sonicator for 1 minute. After that, the flask was stood for 5 minutes and then it was sonicated again for 1 minute under the same condition before. After standing for 5 minutes, the titanium implant was washed twice with purified water. As described above, the titanium implant coated with the growth factors contained in the culture supernatant was obtained. Instead of the culture supernatant, the same treatment was performed by using only DMEM to prepare the non-treated titanium implant material.

**(Example 6)** Implantation example of the sophisticated implant

(1) Patient and the like

The implants prepared in example 5 were implanted in the edentulous jaws of maxillas of eight male from 41 to 68 years old. These patients had no underlying disease which influenced on the bone metabolism such as diabetes, hypertension and the like. Also, they neither drink much amount of alcohol intake nor had any smoking habit.

In one patient, the insertion site was chosen among the maxilla molar region having the distance from a sinus floor not less than 15 mm. Eight implants treated with the growth factor (the treated group) were embedded in one side of the maxilla molar region in the patient, and other 8 implants untreated with the growth factor (the untreated group) were embedded in opposite side of the maxilla molar region in the same patient.

(2) Evaluating method

The implantation results were evaluated by the variation of ISQ (Implant Stability Quotatient) by using Ostell Mentor (Ostell Monitor, Ostell AB, Grothenburug, Sweden) at the immediate after the embedding (time of the embedding), 6 months and 12 months after embedding the 16 implants.

ISQ values changed depending on the bone height or quality around the implant, bonding strength between the bone and the implant, and the like, and they are expressed as a numerical among 1 to 100. In general, when ISQ value is 65 ± 5, the many implant was successfully embedded. The result of the measurement was shown in Fig. 11, and that of the evaluation was shown in following Fig. 8. As shown in Fig. 11, ISQ values of both of the control group and SHED-CM treated group at the implant embedding were in the range. ISQ value of every group increased time-dependently, however, that of SHED-CM treated group was significantly higher at 6 months elapsed ($p<0.05$). The same tendency was shown at 12 months elapsed.

[Table 8]

| Case | | | | |
|---|---|---|---|---|
| Age | Sex | Embedding site | Length of fixture (mm) | Evaluation |
| 45 | male | maxilla molar | 13 | good |
| | | maxilla molar | 13* | normal |
| 41 | male | maxilla molar | 13 | good |
| | | maxilla molar | 11* | normal |
| 45 | male | maxilla molar | 11 | good |
| | | maxilla molar | 11* | normal |
| 56 | male | maxilla molar | 13 | good |
| | | maxilla molar | 13* | normal |
| 40 | male | maxilla molar | 13 | good |
| | | maxilla molar | 13* | normal |
| 68 | male | maxilla molar | 11 | good |
| | | maxilla molar | 13* | normal |
| 50 | male | maxilla molar | 13 | good |
| | | maxilla molar | 11* | normal |
| 57 | male | maxilla molar | 13 | good |
| | | maxilla molar | 13* | normal |
| *: untreated implant AstraTech Implant | | | | |

As shown Fig. 8, in the titanium implant of which surface was coated with the growth factor obtained from immortalized dens deciduous stem cell, progressed unification of the implant with the bone was shown in spite of the length of the fixtures.

**(Example 7)** Analysis of the protein in the culture supernatant and immobilized on the surface of the sophisticated implant

(1) Material and Method

The proteins in the culture supernatant of the stem cell obtained from example 1 were used to be analyzed with LC/MS/MS (MS4 HPLC System (Michrom BioResources Inc.) and LCQ Advantage mass spectrometry system (Thermo Scientific Inc.).

The measurement conditions were as follows.

LC: MS4 HPLC System (Michrom BioResources Inc.)
Column: Magic C18AQ (Michrom BioResources Inc., 0.1 mm (i.d.) x 50 cm)
Eluent: Solution A (2% acetonitrile and 98% water (contains 0.1 % formic acid)), and Solution B (90% acetonitrile and 10% water (contains 0.1 % formic acid)) were used to gradient elution (95% of A: 5% B at 0 min to 0% A: 100% B at 45 min)
Flow rate: 1 μL/min
MS1 : LCQ Advantage mass spectrometry system (Thermo Scientific Inc.)
MS2 : LCQ Advantage mass spectrometry system (Thermo Scientific Inc.)

The proteins immobilized on the implant were subjected to step extraction by using liquid chromatography, wherein 10% EDTA, 4 M of guanidine and 80% acetonitrile were sequentially used. The flow rate of each buffer was set to 2 mL/min to obtain fractions prepared having a protein content at 2 μg/mL. The protein content was determined with Biuret method.

The culture supernatant was precipitated by using both of 100% ethanol and 90% ethanol to be lyophilized, and then prepared so as to be the protein content of 2 μg/mL. The protein content was determined by using Bradford method.

Analyzed results were shown in Table 9.

[Table 9]

| Protein component in SHED-CM (SHED-CM) | Protein component immobilized on the implant (4 M of guanidine) | Accession No. of protein database |
|---|---|---|
| collagen α-1(I) chain | collagen α-1(I) chain* | P02464 |
| collagen α-2(I) chain | collagen α-2(I) chain * | P02452 |
| vimentin | - | P08670 |
| collagen α-1(IV) chain | - | P12109 |
| IGF-binding protein 7 | IGF-binding protein 7 | Q16270 |
| fibronectin | fibronectin* | P02751 |
| decorin | decorin* | P07585 |
| plasminogen activator inhibitor 1 | plasminogen activator inhibitor 1 | P05121 |
| actin, cytoplasmic 2 | actin, cytoplasmic 2 | P60709 |
| sulfhydryl oxidase 1 | sulfhydryl oxidase 1 | 000391 |
| SPARC | - | P09486 |
| metalloproteinase inhibitor 1 | - | P01033 |
| collagen α-1(IV) chain | - | P08123 |
| SCP2 | - | Q9UJQ7 |
| 72 kDa type IV collagenase | - | P08253 |
| B-2 microglobulin | - | AAA51811 |
| Rho GTPase activate protein 18 | - | Q8N392 |
| - | follistatin relating protein 1 | Q12841 |

As shown in Table 9, it was clarified that a lot of protein relating to form cell structure was contained in the culture supernatant of the stem cell obtained in the example 1. Also, since the ration of the adherent cells obtained after culturing the mesenchymal stem cell obtained as mentioned above was high, it was thought that collagen α-1(I) chain, collagen α-2(I) chain, fibronectin and decorin played important roles in the adhesion of the implant and the alveolar bone.

**(Example 8)** Influences by the culture supernatant to cell adherent property and by the lyophilization

(1) Material

The culture supernatant of stem cell obtained in the example 1 was used as the test sample and PBS as the negative control.

(2) Influence to the cell adherent property

Canine bone marrow stroma cells (10 mL of bone marrow fluid was collected from ilium bones of Beagles of 18 to 25 month age, and body weight 15 to 25 kg were preliminary cultured in DMEM containing 10 % FBS, 50 to 150 U/mL of penicillin, and 50 to 150 μg/mL of streptomycin in 5% $CO_2$ incubator at 37 °C in 3 passages) were plated on the purified titanium plate having 15 mm of the diameter at 1.5 to 3 x $10^5$ cell/well (n=3) to investigate the influence to the cell adherent property with or without the culture supernatant and the plasma treatment.

The culture was performed by using the DMEM containing 10% FBS, 50 to 150 U/mL of penicillin and 50 to 150 μg/mL of streptomycin at 37 °C. Prior to the culture, the pure titanium plate with 15 mm diameter was irradiated with atmospheric-pressure plasma (MPS-01K01C, KURITA Seisakusho Co., Ltd.) for 30 seconds at the 10 mm distance from the edge of the plasma, and then the plate was immersed in the culture supernatant or PBS under the conditions of for 24 hours at 37 °C. The adherent cell number at 1 hour and 24 hours after the culture start were released by using trypsin-EDTA, and the floating cells were counted on the hemocytometer. The result was shown in Fig. 12. In Fig 12, N-PBS shows that the incubation was performed in PBS without plasma treatment, P-PBS was done in PBS with plasma treatment, N-CM was done in the culture supernatant without plasma treatment and P-CM was done in the culture supernatant with plasma treatment.

In N-PBS, the adherent cell number was not increased greatly even after 24 hours, however in P-PBS, the number was increased greatly. Also, in the both of the N-CM group and P-CM group, the adherent cell number at 24 hours after treatment was increased greatly.

There was the significant difference between the adherent cell number of the N-PBS group and that of P-CM group at 24 hours after the treatment ($p < 0.01$). There was also the significant difference between the adherent cell number of the P-PBS group and that of P-CM group ($p < 0.05$).

Further, the cells after 24 hours were stained with DAPI and phalloidin, and the condition of the adherent cells was observed by using the fluorescence microscopy (Al+, Nikon Corp.) of 250 magnifications (the excitation wavelength: 358 nm (DAPI), 560 nm (phalloidin), the emission wavelength: 465 nm (DAPI), 575 nm (phalloidin)).

The result was shown in Fig. 13. As shown Fig. 13, the cell growth was clearly increased in both of the N-CM group and P-CM group.

(3) Influence to cell proliferation with lyophilization

One mL of the culture supernatant was dispensed in the plastic tube with a screw cap (the content volume 2 mL, Corning Inc.) and stored at 4 °C, -15 °C and -80 °C for 1 week, 2 weeks and 1 month respectively.

The influence was confirmed by using bromouridine assay. As an assay kit, BrdU labeling and detection kit II (Roche Applied Science) was used, and the assay was performed according to the attached instruction.

The mesenchymal stem cell obtained as the same as in the example 1 was plated in 96 well plate at $1 \times 10^5$ cell/well. The stored culture supernatant as mentioned above was added in the plate at 100 μL/well and the cell was cultured in the 5% $CO_2$ incubator for 24 hours at 37 °C. After that, the growth of the stem cell was observed and the growth ratio was calculated.

The result was shown in Fig. 14.

When the cell was stored at -80 °C, the activity of the stored supernatant for 1 month decreased compared to that for 1 week; however there was no significant difference between them. On the other hand, there was the significant difference between the stored supernatant for 2 weeks at -80 °C and that for 1 month at -15 °C ($P<0.05$). Also, there was the significant difference between the stored supernatant for 2 weeks at -80 °C and that for 1 month at 4 °C ($P < 0.05$). There was also the significant difference between the stored supernatant for 1 week at - 80 °C and that for 2 weeks at 4 °C ($P < 0.05$).

From the above, it is thought that the storage life of the supernatant is for 1 month at -80 °C, for 1 month at -15 °C and 2 weeks at 4 °C.

Consequently, it was indicated that the growth of the stem cell which produces the growth factor was promoted to strengthen the adhesion between the implant and the bone to which the implant was embedded.

**Industrial Applicability**

The present invention is quite useful in the medical and dental fields.

**Free Text of Sequence Listing**

Sequence number 1: a forward primer for ALP
Sequence number 2: a reverse primer for ALP
Sequence number 3: a forward primer for OCN
Sequence number 4: a reverse primer for OCN
Sequence number 5: a forward primer for OPN
Sequence number 6: a reverse primer for OPN
Sequence number 7: a forward primer for BSP
Sequence number 8: a reverse primer for BSP
Sequence number 9: a forward primer for Collagen 1

Sequence number 10: a reverse primer for Collagen 1
Sequence number 11: a forward primer for GAPDH
Sequence number 12: a reverse primer for GAPDH
Sequence number 13: a collagen $\alpha$-1(I) chain precursor
Sequence number 14: a collagen $\alpha$-1(II) chain precursor
Sequence number 15: a collagen $\alpha$-1(XXVII) chain precursor
Sequence number 16: a collagen $\alpha$-2(I) chain precursor
Sequence number 17: fibronectin
Sequence number 18: fibronectin
Sequence number 19: decorin
Sequence number 20: decorin
Sequence number 21: osteocalcin
Sequence number 22: osteopontin
Sequence number 23: bone sialoprotein 2
Sequence number 24: vesicular endothelial growth factor

**Claims**

**1.** A preparation method for cell product comprising the steps of:

preliminary culturing 5 x $10^3$ to 5 x $10^4$ cell/mL of the bone marrow stromal cells or immortalized dens deciduous stem cells in a medium containing 5 to 15% serum, 50 to 150 U/mL of penicillin and 50 to 150 μg/mL of streptomycin at a temperature from 34 to 37.5 °C in the presence of 5% $CO_2$;
selecting the cell having both of a prescribed shape and adhesive property; and
culturing the cells selected in said selecting step under the same condition as that of the preliminary culturing to collect a culture supernatant after 40 to 56 hours from start of the culture.

**2.** The preparation method for cell product according to the claim 1, wherein said bone marrow stromal cell is derived from the any one of cell selected from the group comprising of rat femur, swine dental pulp and human dens deciduous.

**3.** The preparation method for cell product according to the claim 2, wherein said cell is incubated at 37 °C.

**4.** The preparation method for cell product according to the claim 3, wherein said prescribed shape is spindle shape and fusiform.

**5.** The preparation method for cell product according to the claim 1, wherein said culture supernatant is collected after 44 to 52 hours from the start of the culture.

**6.** The preparation method for cell product according to the claim 5, wherein said culture supernatant is collected after 48 hours from the start of the culture.

**7.** The preparation method for cell product according to the claim 1, wherein said culture medium comprises at least one of the medium selected from the group comprising of Dulbecco's medium, Iscove's modified Dulbecco's Medium, Ham's F12 medium and RPMI1640.

**8.** The preparation method for cell product according to the claim 1, wherein said serum is at least one selected from the group consisting of fetal bovine serum, bovine serum, horse serum, human serum, and sheep serum.

**9.** The preparation method for cell product according to the claim 1, wherein said culture medium comprises 10% of the fetal bovine serum, 100 U/mL of penicillin and 100 μg /mL of streptomycin.

**10.** The preparation method for cell product according to the any one of the claims 1 to 9, further comprising the step of;
preparing a precipitate by performing centrifugation and ethanol precipitation treatment; and
lyophilizing the precipitate obtained from said preparation step of precipitate.

**11.** A cell product prepared by the method according to any one of the claim 1 to 10.

**12.** The cell product according to the claim 11, wherein said culture supernatant comprises at least one of type I collagen (Col-I), fibronectin, decorin and vascular endothelial growth factor (VEGF).

**13.** The cell product according to the claim 12, wherein said culture supernatant further comprises insulin-like growth factor - binding protein 7, follistatin related protein, metalloproteinase inhibitor I, tissue plasminogen activator inhibitor, actin, and sulfhydryl oxidase I.

**14.** The cell product according to the claim 13, wherein said culture supernatant further comprises SPARC, collagen α2 (IV) chain, β-2-microglobulin and Rho GTPase-activating protein 18.

**15.** A production method for sophisticated implant comprising the steps of:

washing a surface of an implant material with an organic solvent to prepare a washed implant material;
immersing said washed implant material in a first calcification solution having prescribed composition at prescribed temperature for 3 to 6 hours to prepare an immersed implant material; and
incubating said immersed implant material in a second calcification solution containing the cell product at a prescribed concentration according to any of the claims 12 to 14 for 2 to 5 days.

**16.** The production method for the sophisticated implant according to the claim 16, wherein said implant material is formed by any material selected from the group consisting of titanium, zirconia, hydroxyapatite and β-TCP.

**17.** The production method for sophisticated implant according to the claim 16, wherein said organic solvent is acetone and 60 to 80% of ethanol.

**18.** The production method for the sophisticated implant according to the claim 16, wherein said first calcification solution is composed of 100 to 150 mM of NaCl, 2.5 to 3.5 mM of $CaCl_2 \cdot H_2O$, 1.5 to 2.5 mM of $K_2HPO_4$ and 25 to 75 mM of Tris-HCl (pH 7.2 to 7.6), and said second calcification solution is composed of 130 to 160 mM of $KNO_3$, 1 to 2 mM of $Ca(NO_3)_2 \cdot 4H_2O$ and 0.5 to 1.5 mM of $K_2HPO_4$ (pH 7.4).

**19.** The production method for the sophisticated implant according to the claim 18, wherein said first calcification solution is composed of 136.8 mM of NaCl, 3.10 mM of $CaCl_2 \cdot H_2O$, 1.86 mM of $K_2HPO_4$, and 50 mM of Tris-HCl (pH 7.4), and said second calcification solution is composed of 142.8 mM of $KNO_3$, 1.5 mM of $Ca(NO_3)_2 \cdot 4H_2O$, and 0.9 mM of $K_2HPO_4$ (pH 7.4).

**20.** The production method for the sophisticated implant according to the claim 16, wherein said prescribed temperature is from 36 to 38 °C and the prescribed immersing time is 4 hours.

**21.** The production method for the sophisticated implant according to the claim 15, wherein said cell product of described concentration is at a concentration of 5 to 20 mg/mL.

**22.** A sophisticated implant produced by the method according to any one of the claims 15 to 21.

**23.** The sophisticated implant according to the claim 22, wherein said implant has at least type I collage, fibronectin and decorin on the surface.

**24.** The sophisticated implant according to the claim 23, wherein said implant further comprises at least one of protein selected from the group consisting of insulin-like growth factor - binding protein 7, follistatin related protein, metalloproteinase inhibitor I, tissue plasminogen activator inhibitor, actin and sulfhydryl oxidase I on the surface of the implant.

**25.** The sophisticated implant according to the claim 22, wherein said sophisticated implant is formed by a material selected from the group consisting of titanium, zirconia, hydroxyapatite and β-TCP.

Fig. 1

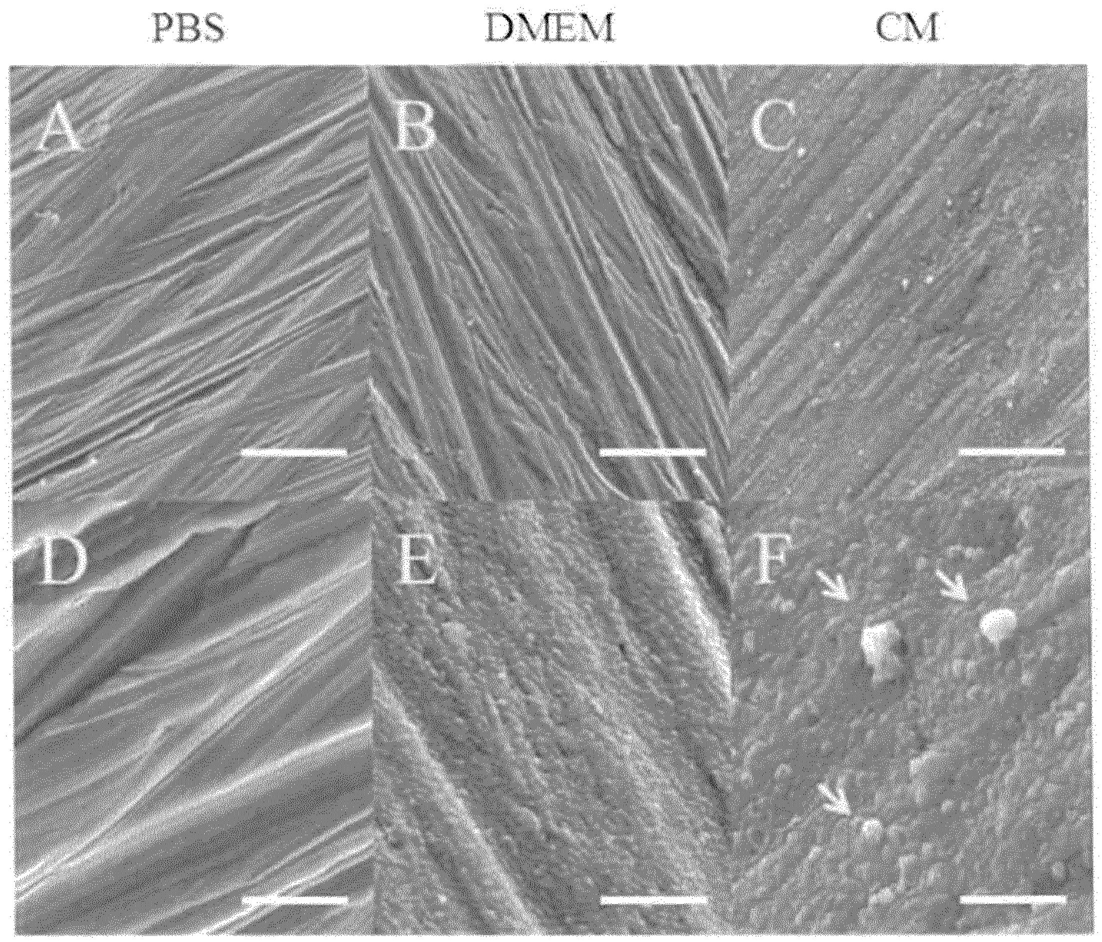

Fig. 2

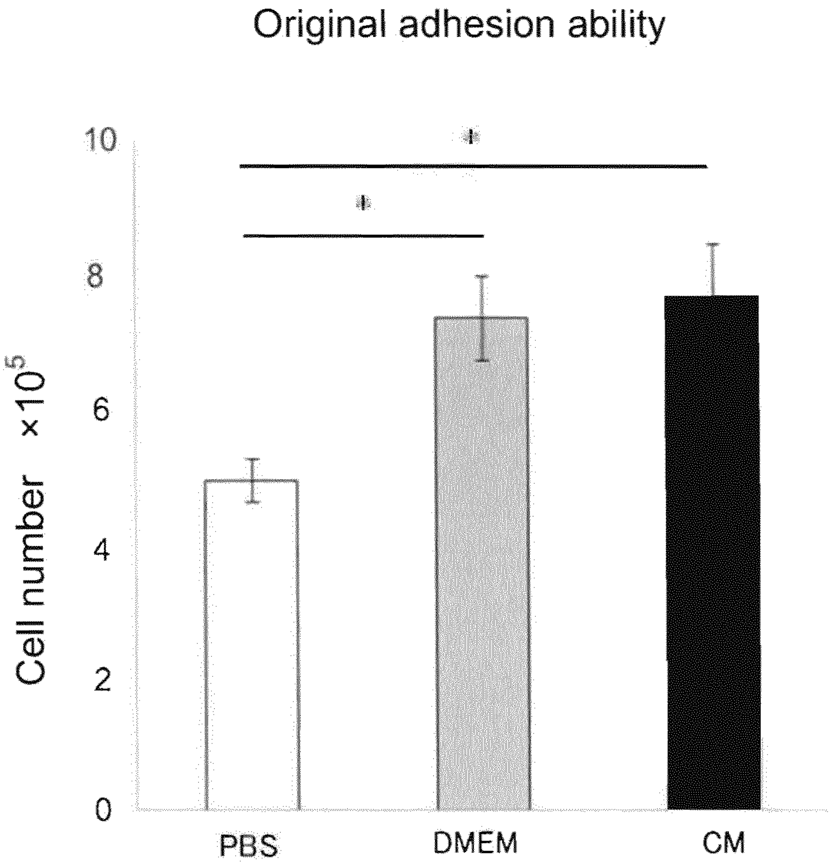
Original adhesion ability
10
8
6
4
2
0
Cell number ×10^5
*
*
PBS
DMEM
CM

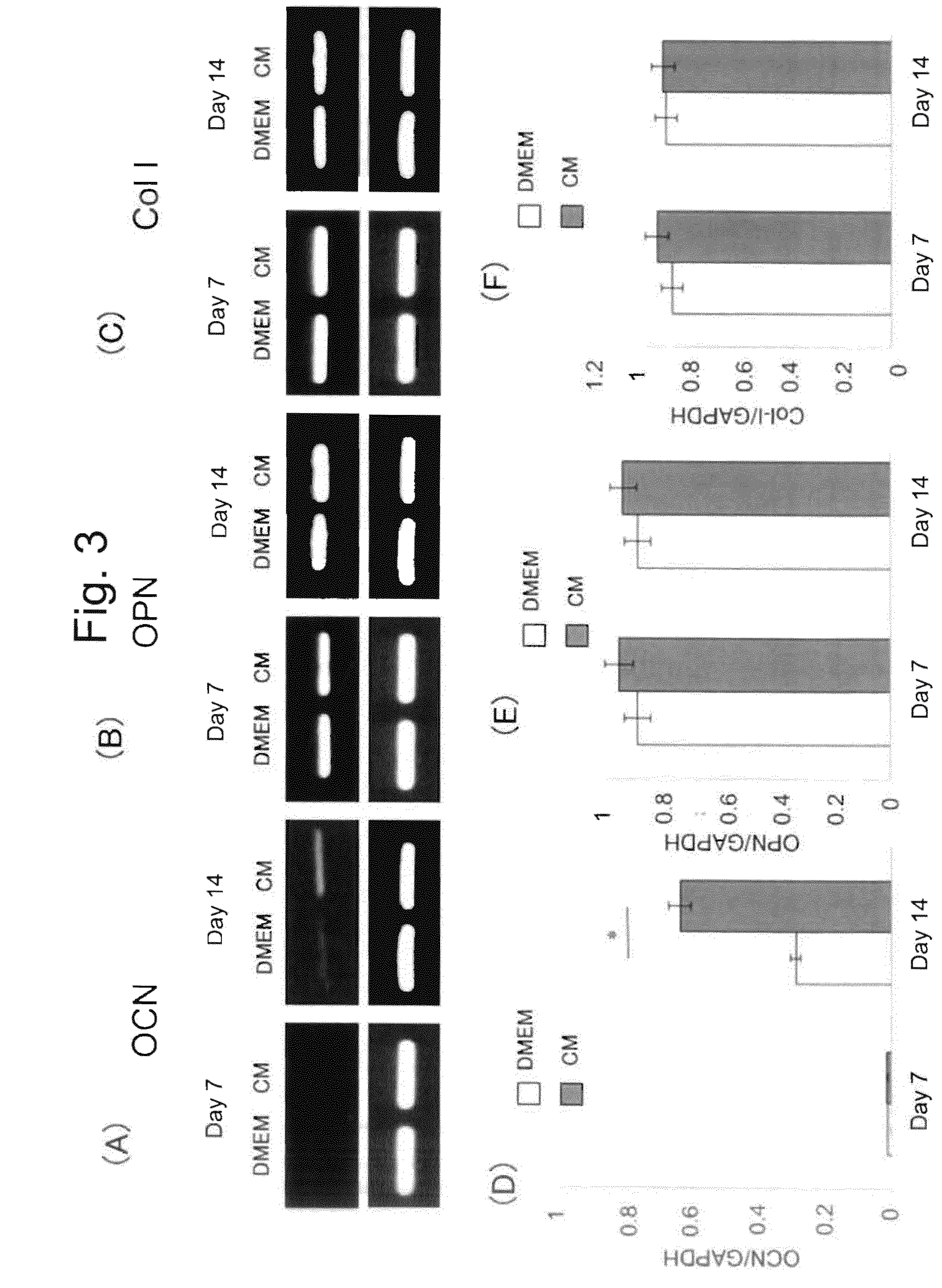
(A)
OCN
(B)
OPN
(C)
Col I
Day 7
Day 14
DMEM
CM
(D)
OCN/GAPDH
0
0.2
0.4
0.6
0.8
1
*
(E)
OPN/GAPDH
(F)
Col-I/GAPDH
1.2

Fig. 3

Fig. 4

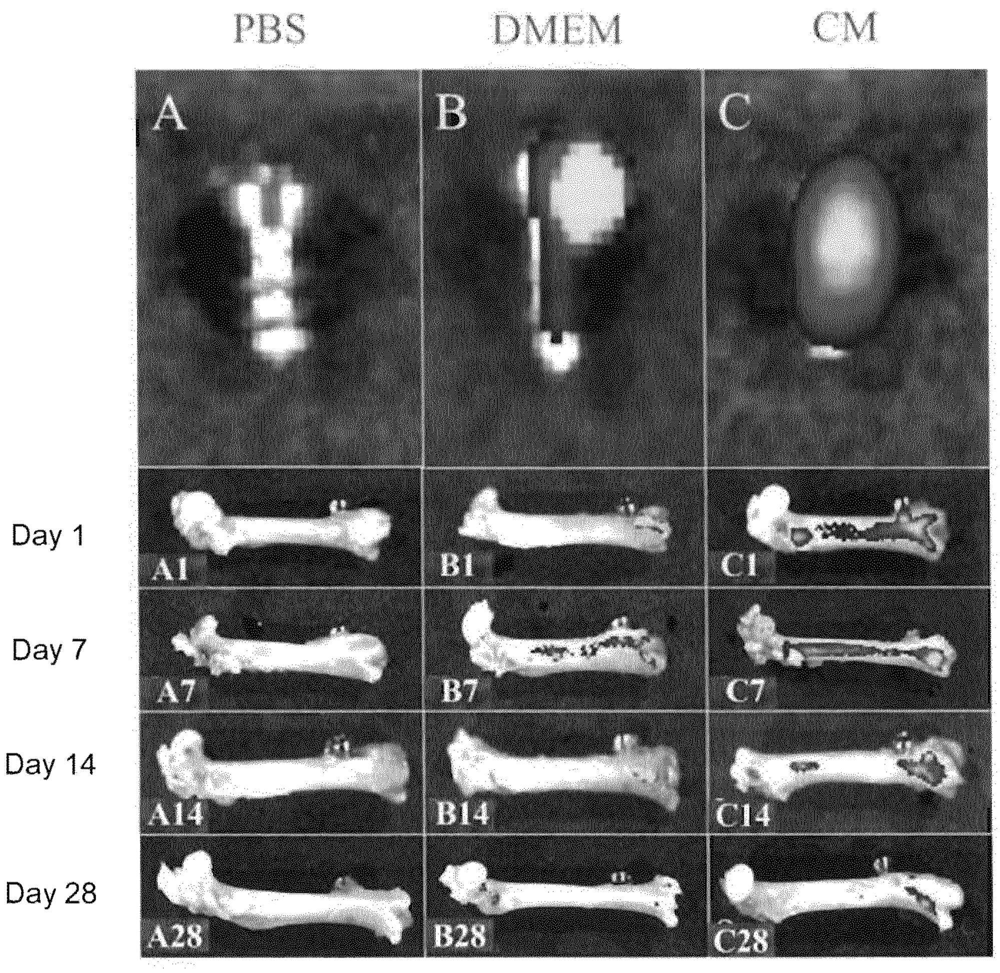
PBS
DMEM
CM
A
B
C
Day 1
Day 7
Day 14
Day 28
A1
B1
C1
A7
B7
C7
A14
B14
C14
A28
B28
C28

Fig. 5

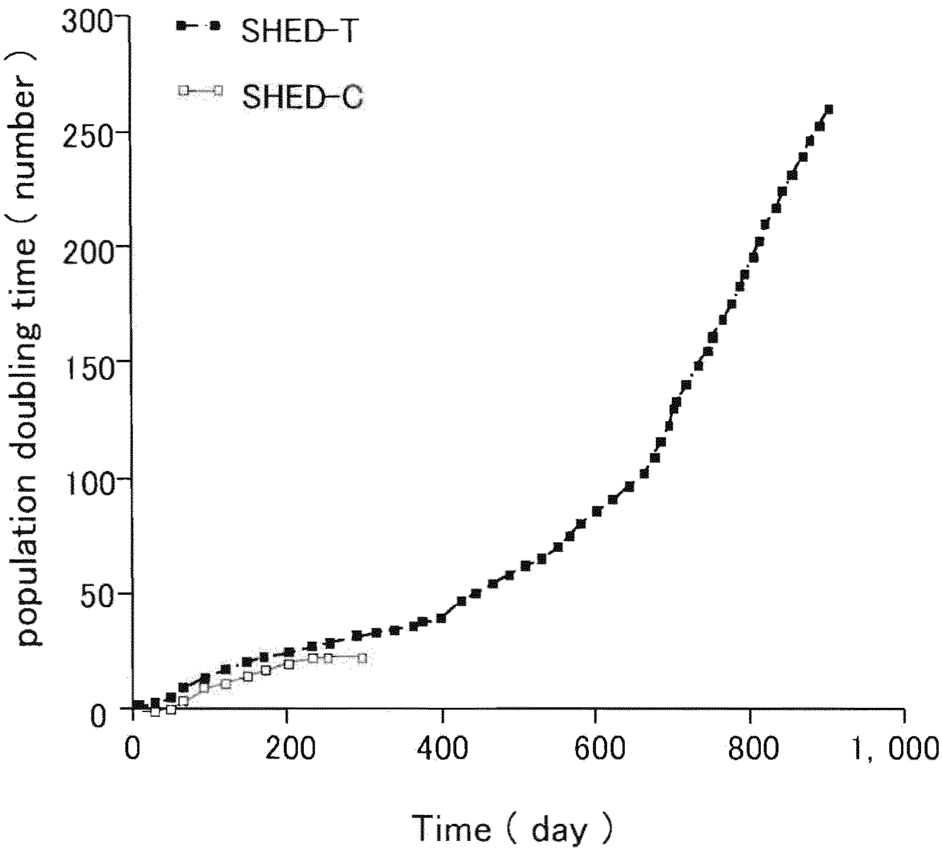
SHED-T
SHED-C
population doubling time ( number )
300
250
200
150
100
50
0
0
200
400
600
800
1, 000
Time ( day )

Fig. 6

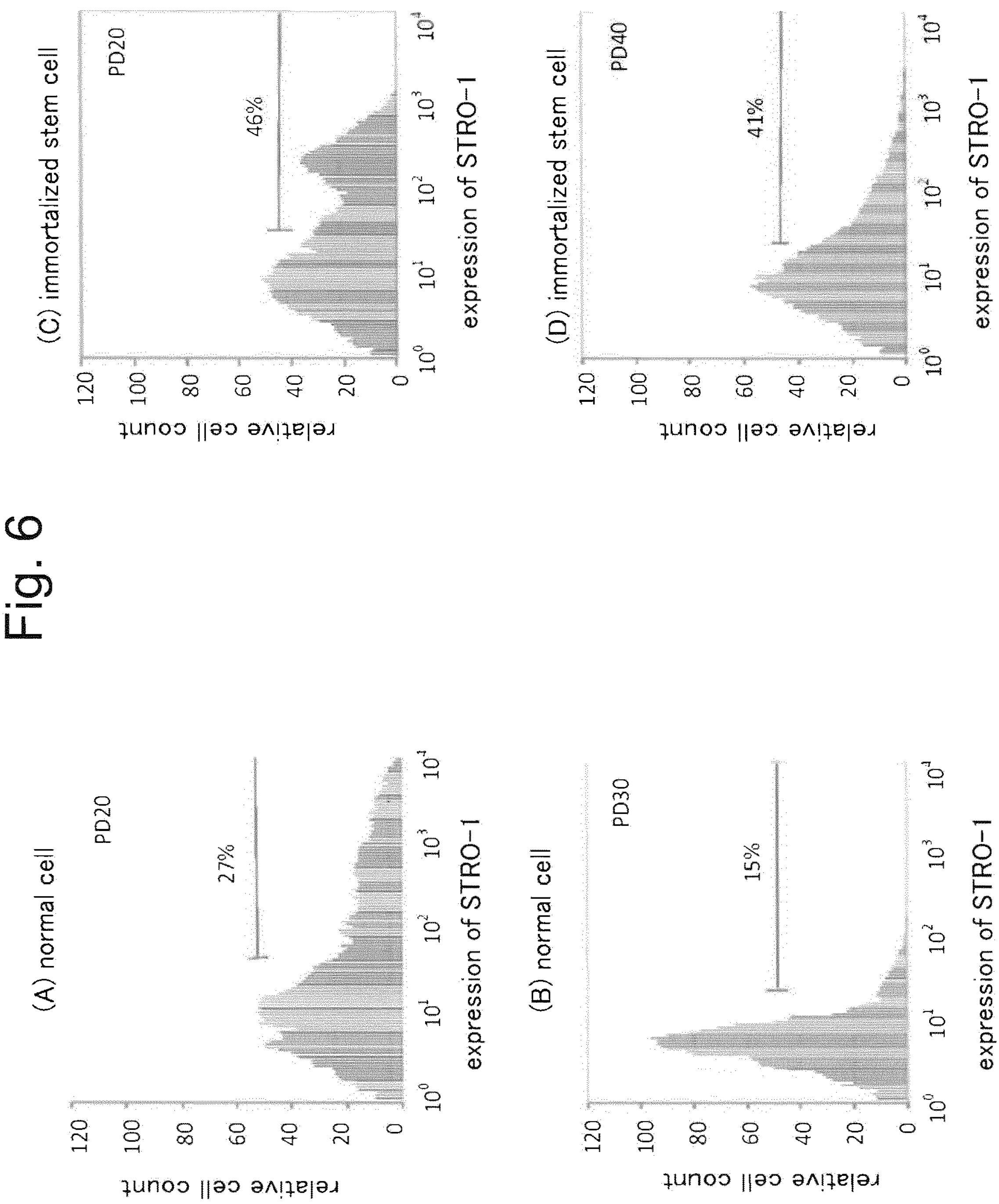
(A) normal cell
PD20
27%
(B) normal cell
PD30
15%
(C) immortalized stem cell
PD20
46%
(D) immortalized stem cell
PD40
41%
relative cell count
expression of STRO-1
0
20
40
60
80
100
120
$10^0$
$10^1$
$10^2$
$10^3$
$10^4$

Fig. 7

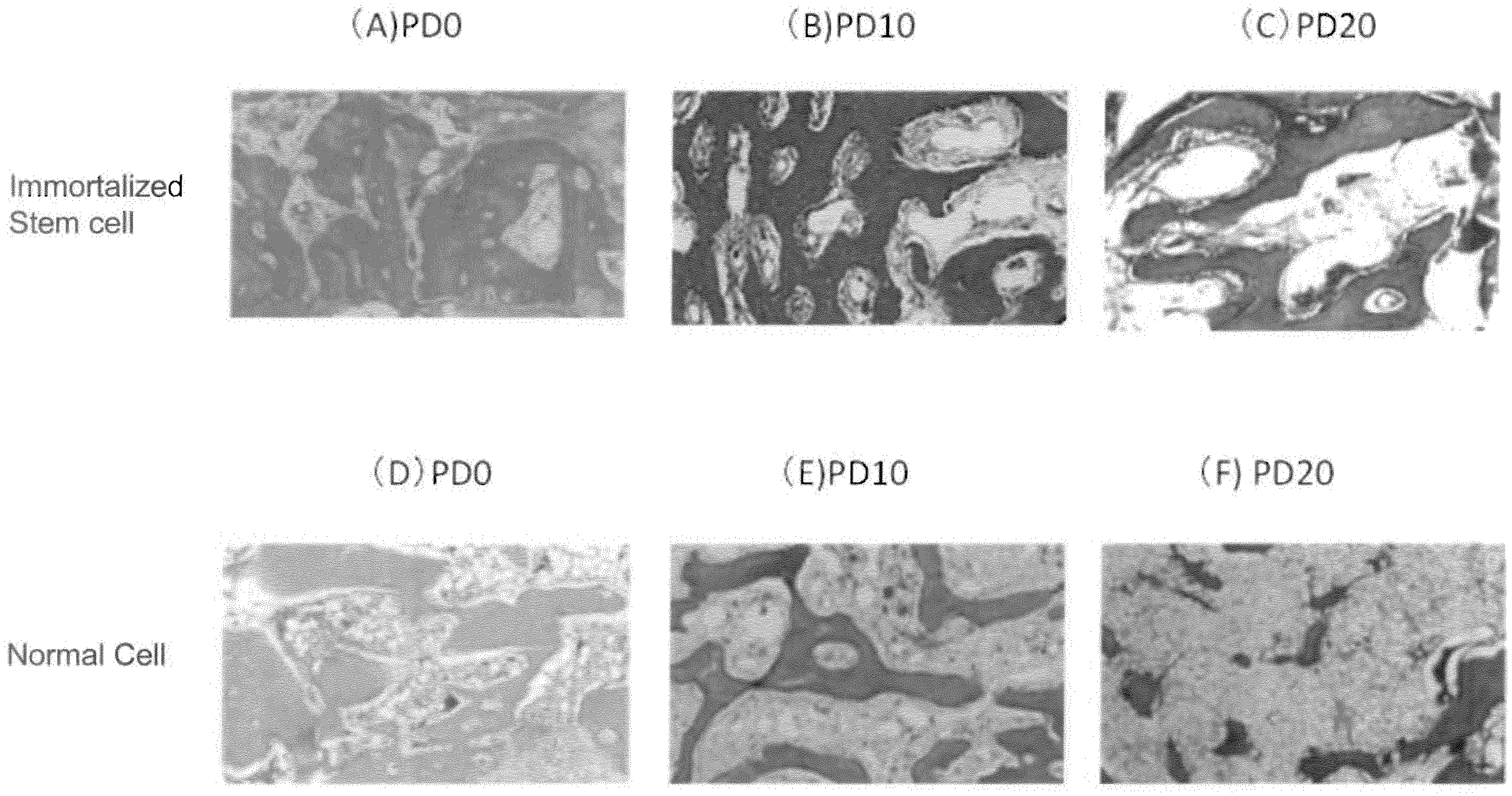


(stained with HE ×200)

Fig. 8

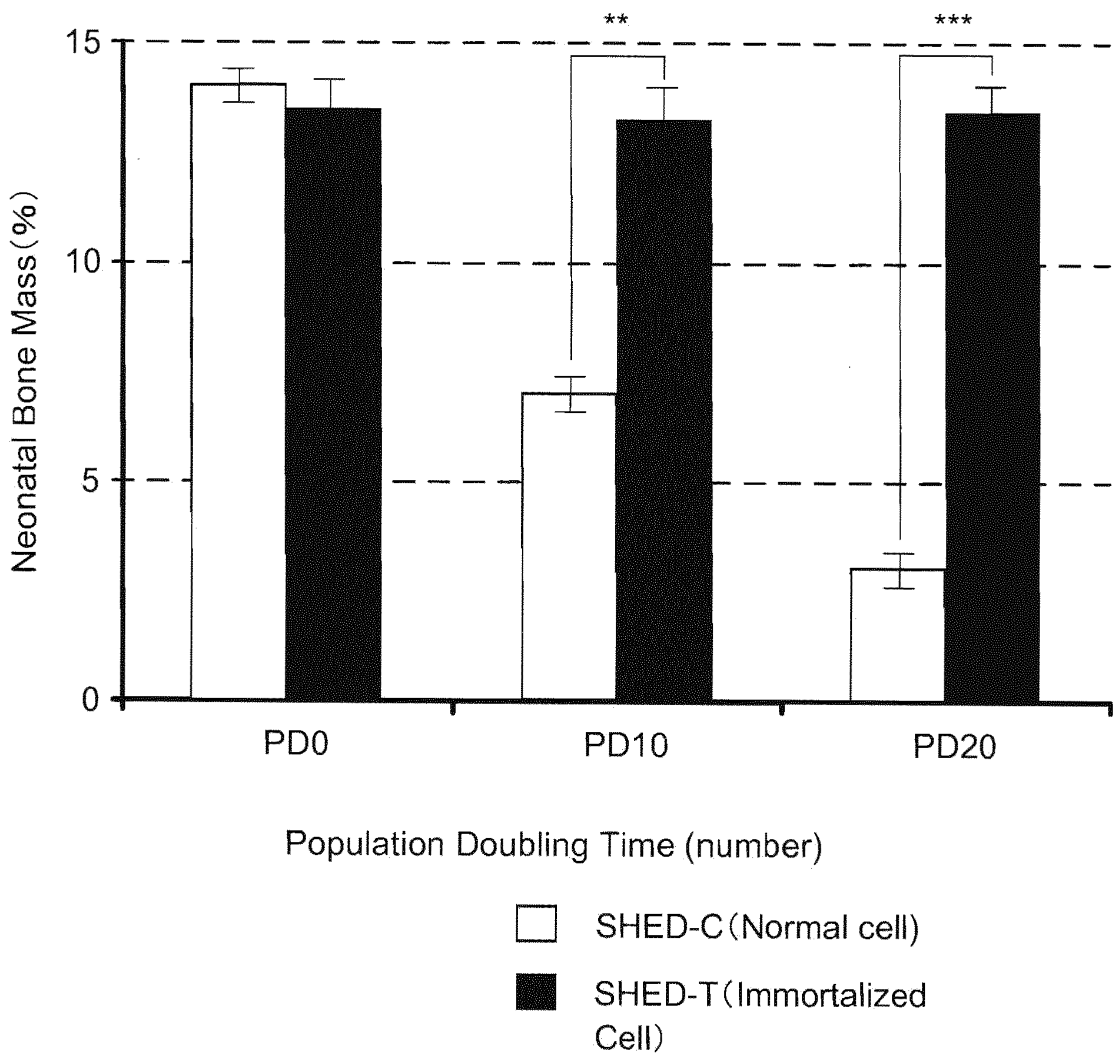

15
10
5
0
**
***
Neonatal Bone Mass(%)
PD0
PD10
PD20
Population Doubling Time (number)
SHED-C(Normal cell)
SHED-T(Immortalized Cell)

Fig. 9

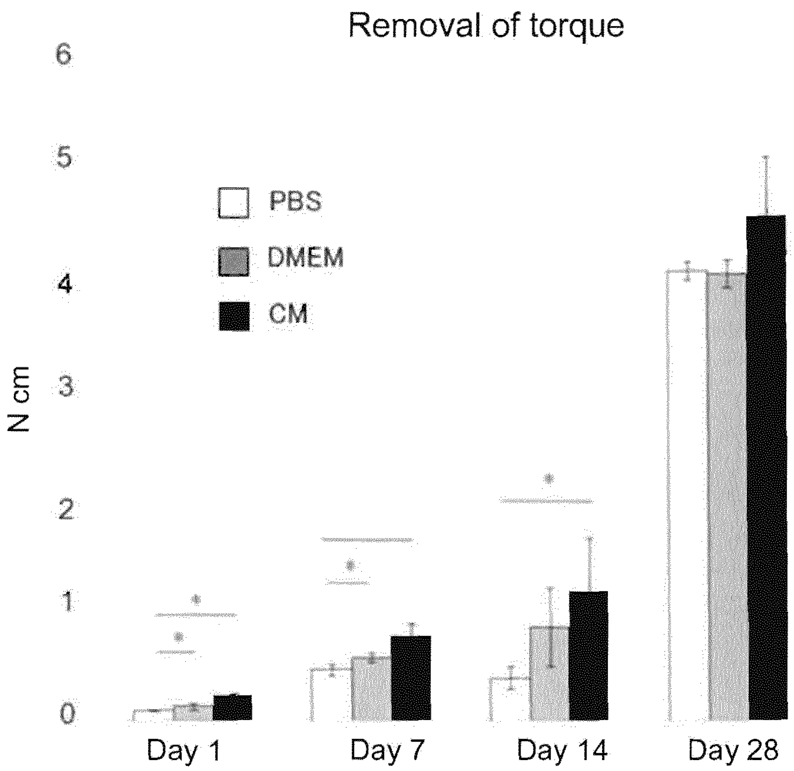
Removal of torque
6
5
4
3
2
1
0
N cm
PBS
DMEM
CM
*
*
*
*
Day 1
Day 7
Day 14
Day 28

Fig. 10

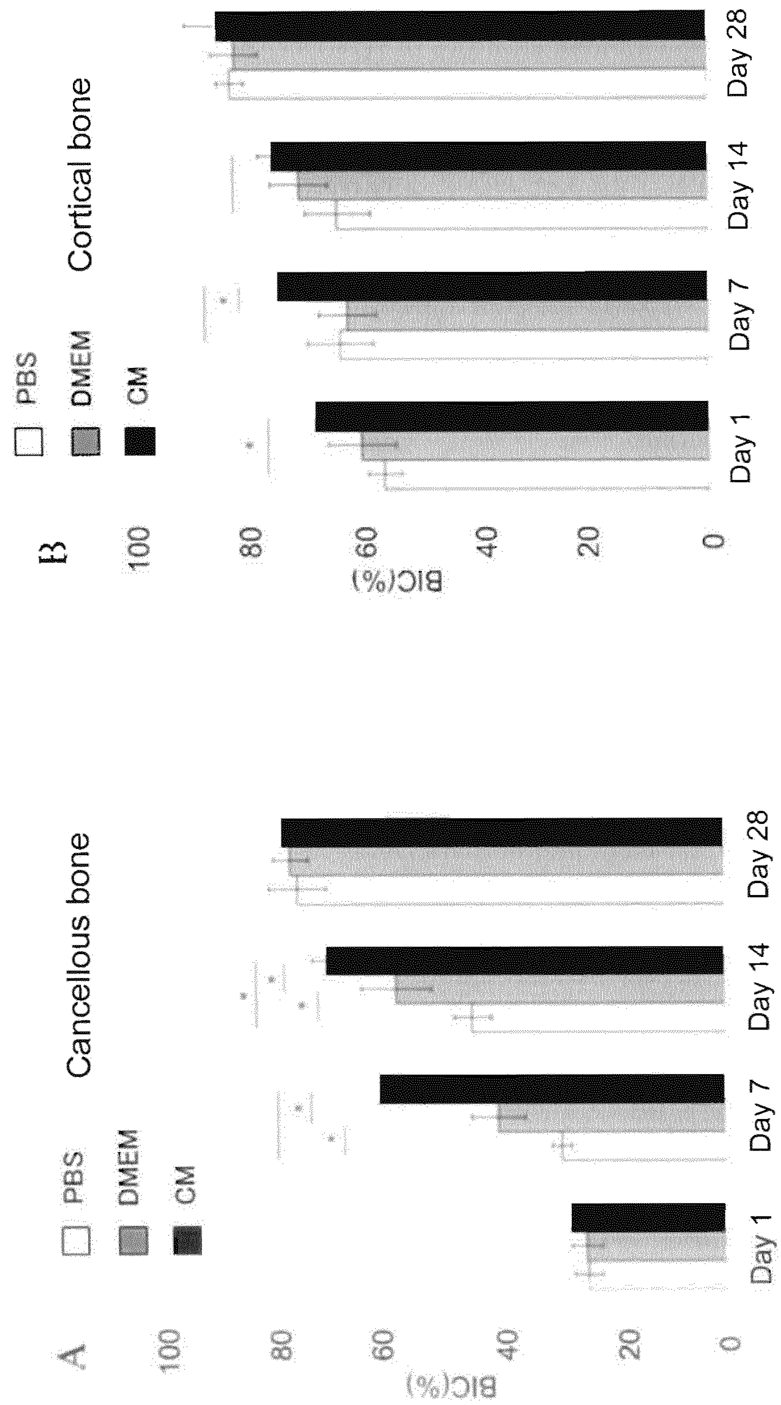
A
Cancellous bone
B
Cortical bone
PBS
DMEM
CM
BIC(%)
100
80
60
40
20
0
Day 1
Day 7
Day 14
Day 28

Fig. 11

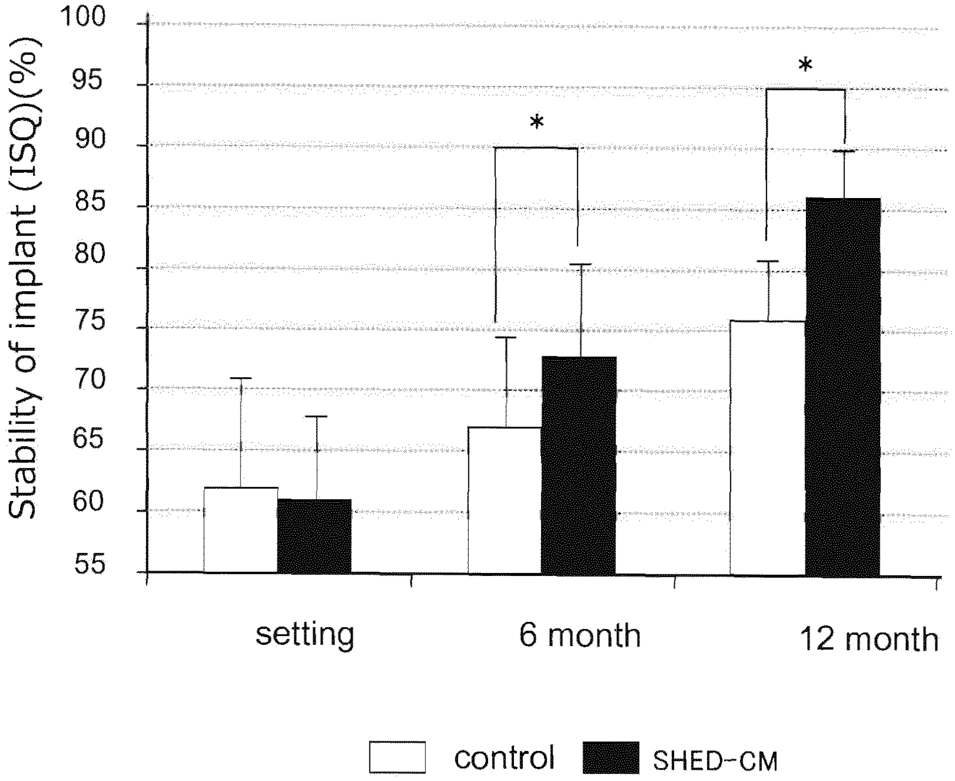
Stability of implant (ISQ)(%)
100
95
90
85
80
75
70
65
60
55
*
*
setting
6 month
12 month
control
SHED-CM

Fig. 12

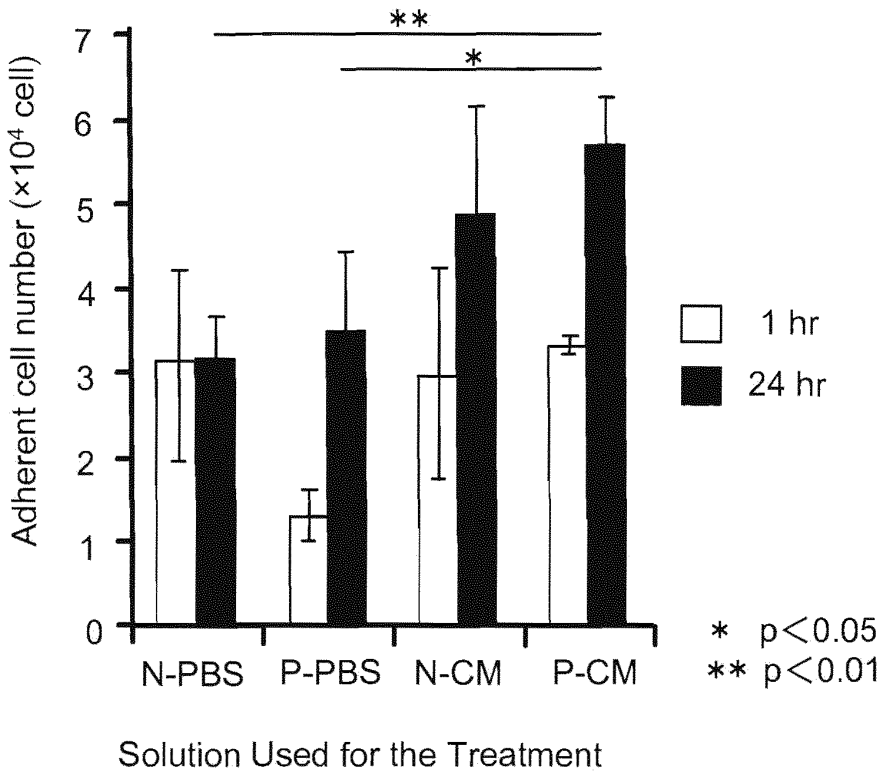
**
*
7
6
5
4
3
2
1
0
Adherent cell number (×10^4 cell)
1 hr
24 hr
N-PBS
P-PBS
N-CM
P-CM
* p<0.05
** p<0.01
Solution Used for the Treatment

Fig. 13

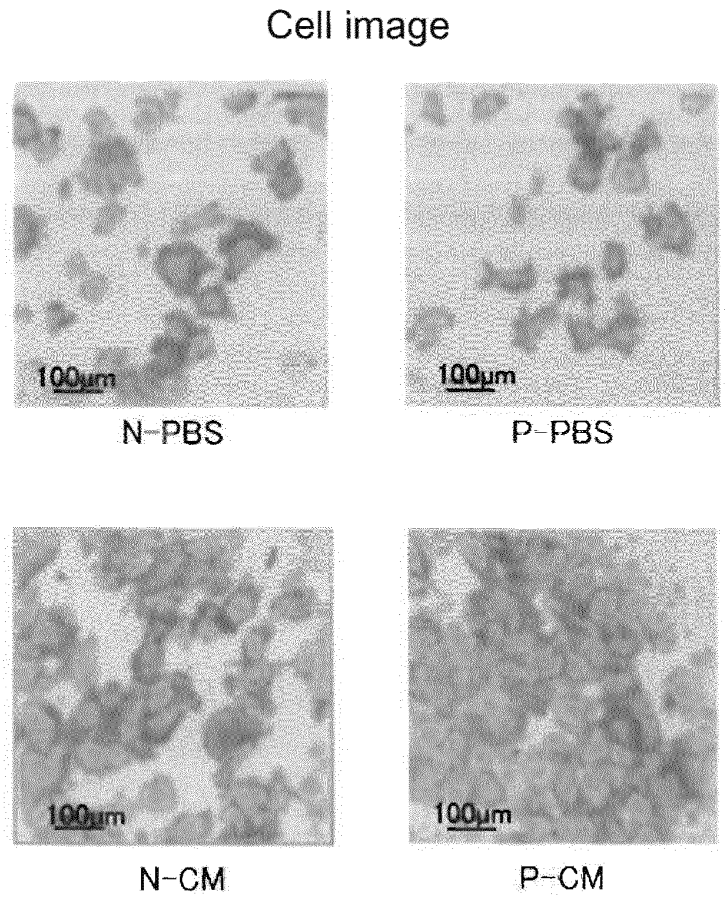

Fig. 14

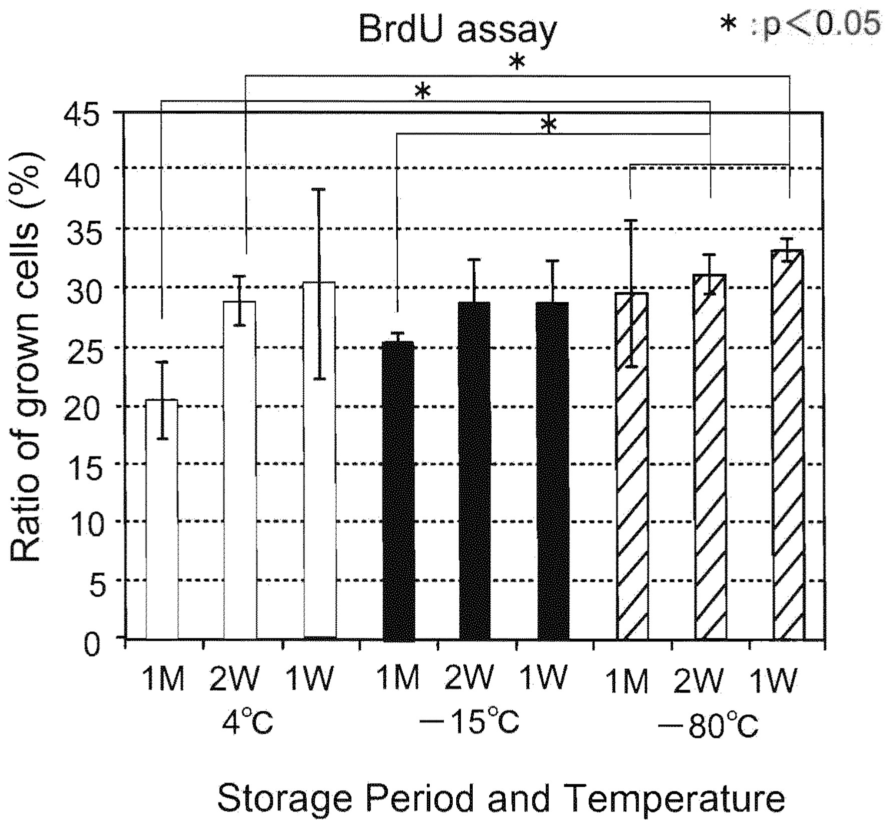
BrdU assay
* :p<0.05
*
*
*
Ratio of grown cells (%)
45
40
35
30
25
20
15
10
5
0
1M 2W 1W
4°C
1M 2W 1W
−15°C
1M 2W 1W
−80°C
Storage Period and Temperature

## INTERNATIONAL SEARCH REPORT

International application No. PCT/JP2013/084990

A. CLASSIFICATION OF SUBJECT MATTER
*C12P21/00*(2006.01)i, *A61L27/00*(2006.01)i, *C07K14/47*(2006.01)i, *C07K14/475*(2006.01)i, *C07K14/65*(2006.01)i, *C07K14/78*(2006.01)i, *C12N5/071*(2010.01)n, *C12N15/09*(2006.01)n

According to International Patent Classification (IPC) or to both national classification and IPC

B. FIELDS SEARCHED

Minimum documentation searched (classification system followed by classification symbols)
C12P21/00, A61L27/00, C07K14/47, C07K14/475, C07K14/65, C07K14/78, C12N5/071, C12N15/09

Documentation searched other than minimum documentation to the extent that such documents are included in the fields searched

| | | | |
|---|---|---|---|
| Jitsuyo Shinan Koho | 1922-1996 | Jitsuyo Shinan Toroku Koho | 1996-2014 |
| Kokai Jitsuyo Shinan Koho | 1971-2014 | Toroku Jitsuyo Shinan Koho | 1994-2014 |

Electronic data base consulted during the international search (name of data base and, where practicable, search terms used)
CAplus/MEDLINE/BIOSIS/WPIDS(STN), JSTPlus/JMEDPlus/JST7580(JDreamIII)

C. DOCUMENTS CONSIDERED TO BE RELEVANT

| Category* | Citation of document, with indication, where appropriate, of the relevant passages | Relevant to claim No. |
|---|---|---|
| X/Y/A | LIU K. et al., The interactions between brain microvascular endothelial cells and mesenchymal stem cells under hypoxic conditions, Microvasc. Res., 2008.01, Vol.75, No.1, p.59-67 | 11-14/1-14/15-25 |
| Y/A | WO 2011/118795 A1 (Nagoya University), 29 September 2011 (29.09.2011), paragraphs [0026], [0084]; examples & US 2013/0195991 A1 & EP 2554175 A1 | 1-14/15-25 |
| Y/A | SAKAI K. et al., Human dental pulp-derived stem cells promote locomotor recovery after complete transection of the rat spinal cord by multiple neuro-regenerative mechanisms, J. Clin. Invest., 2012.01.03, Vol.122, No.1, p.80-90 | 1-14/15-25 |

☒ Further documents are listed in the continuation of Box C.  ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 03 March, 2014 (03.03.14) | 11 March, 2014 (11.03.14) |

| Name and mailing address of the ISA/ | Authorized officer |
|---|---|
| Japanese Patent Office | |
| Facsimile No. | Telephone No. |

Form PCT/ISA/210 (second sheet) (July 2009)

**INTERNATIONAL SEARCH REPORT**

International application No. PCT/JP2013/084990

C (Continuation). DOCUMENTS CONSIDERED TO BE RELEVANT

| Category* | Citation of document, with indication, where appropriate, of the relevant passages | Relevant to claim No. |
|---|---|---|
| Y/A | OSUGI M. et al., Conditioned media from mesenchymal stem cells enhanced bone regeneration in rat calvarial bone defects, Tissue Eng. Part A, 2012.07, Vol.18, No.13-14, p.1479-1489 | 1-14/15-25 |
| Y/A | MA M. et al., Mesenchymal stromal cells may enhance metastasis of neuroblastoma via SDF-1/CXCR4 and SDF-1/CXCR7 signaling, Cancer Lett., 2011.12.15, Vol.312, No.1, p.1-10 | 1-14/15-25 |
| A | WO 00/21456 A1 (SOCIETE ANONYME NATURAL IMPLANT), 20 April 2000 (20.04.2000), abstract & US 2001/0055745 A1 & EP 1121064 A & JP 2002-527139 A | 1-25 |
| A | FERGUSON S.J. et al., Biomechanical comparison of different surface modifications for dental implants, Int. J. Oral. Maxillofac. Implants, 2008.12, Vol.23, No.6, p.1037-1046 | 1-25 |
| A | LI L. et al., Paracrine action mediate the antifibrotic effect of transplanted mesenchymal stem cells in a rat model of global heart failure, Mol. Biol. Rep., 2009.04, Vol.36, No.4, p.725-731 | 1-25 |
| A | PSALTIS P.J. et al., Enrichment for STRO-1 expression enhances the cardiovascular paracrine activity of human bone marrow-derived mesenchymal cell populations, J. Cell. Physiol., 2010.05, Vol.223, No.2, p.530-540 | 1-25 |

Form PCT/ISA/210 (continuation of second sheet) (July 2009)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 2012509750 A
- JP 2004531461 A

SEQUENCE LISTING

<110>  Quarrymen Corporation
Quarrymen Corporation

<120>  Highly-functional implant

<130>  P13YY008PCT

<150>  JP2012-282001
<151>  2012-12-26

<160>  24

<170>  PatentIn version 3.5

<210>  1
<211>  20
<212>  DNA
<213>  Homo sapiens

<400>  1
gctgtgaagg gcttcttgtc    20

<210>  2
<211>  20
<212>  DNA
<213>  Homo sapiens

<400>  2
cgcctatcag ctaatgcaca    20

<210>  3
<211>  20
<212>  DNA
<213>  Homo sapiens

<400>  3
tgaggaccct ctctctgctc    20

<210>  4
<211>  20
<212>  DNA
<213>  Homo sapiens

<400>  4
gagctcacac acctccctgt    20

<210>  5
<211>  20
<212>  DNA
<213>  Homo sapiens

<400>  5
aggtcctcat ctgtggcatc    20

<210>  6
<211>  20

<212> DNA
<213> Homo sapiens

<400> 6
agactggcag tggtttgctt 20

<210> 7
<211> 19
<212> DNA
<213> Homo sapiens

<400> 7
ttctcgagaa aaatttcca 19

<210> 8
<211> 20
<212> DNA
<213> Homo sapines

<400> 8
tcactggtgg tagtaataat 20

<210> 9
<211> 20
<212> DNA
<213> Homo sapiens

<400> 9
cgatgccatt ttctccctta 20

<210> 10
<211> 20
<212> DNA
<213> Homo sapiens

<400> 10
ctctggtacc gctggagaag 20

<210> 11
<211> 20
<212> DNA
<213> Homo sapiens

<400> 11
atgactctac ccacggcaag 20

<210> 12
<211> 20
<212> DNA
<213> Homo sapiens

<400> 12
ttcagctctg ggatgacctt 20

<210> 13
<211> 18

<212> PRT
<213> Homo sapiens

<400> 13

Ser Gly Glu Tyr Trp Ile Asp Pro Asn Gln Gly Cys Asn Leu Asp Ala
1 5 10
15

Ile Lys

<210> 14
<211> 40
<212> PRT
<213> Homo sapiens

<400> 14

Asp Gly Glu Pro Gly Thr Pro Gly Asn Pro Gly Pro Pro Gly Pro Pro
1 5 10
15

Gly Pro Pro Gly Pro Pro Gly Leu Gly Gly Gly Asn Phe Ala Ala Gln
20 25
30

Met Ala Gly Gly Phe Asp Glu Lys
35 40

<210> 15
<211> 19
<212> PRT
<213> Homo sapiens

<400> 15

Asp Gly Tyr Pro Gly Pro Leu Gly Leu Asp Gly Lys Pro Gly Leu Pro
1 5 10
15

Gly Pro Lys

<210> 16
<211> 32
<212> PRT
<213> Homo sapiens

<400> 16

Gly Pro Asn Gly Asp Ala Gly Arg Pro Gly Glu Pro Gly Leu Met Gly
1 5 10
15

Pro Arg Gly Leu Pro Gly Ser Pro Gly Asn Val Gly Pro Ala Gly Lys
20 25
30

<210> 17
<211> 11
<212> PRT
<213> Homo sapiens

<400> 17

Asp Leu Gln Phe Val Glu Val Thr Asp Val Lys
1 5 10

<210> 18
<211> 15
<212> PRT
<213> Homo sapiens

<400> 18

Val Asp Val Leu Pro Val Asn Leu Pro Gly Glu His Gly Gln Arg
1 5 10
15

<210> 19
<211> 11
<212> PRT
<213> Homo sapiens

<400> 19

Ile Ser Pro Glu Ala Phe Lys Pro Leu Val Lys
1 5 10

<210> 20
<211> 14
<212> PRT
<213> Homo sapiens

<400> 20

Thr Ser Tyr Thr Ala Val Ser Leu Tyr Ser Asn Pro Val Arg
1 5 10

<210> 21
<211> 47
<212> PRT
<213> Homo sapiens

<400> 21

Ser Leu Leu Pro Leu Ala Ile Leu Ala Ala Leu Ala Val Ala Ala Leu
1 5 10
15

Cys Tyr Glu Ser His Glu Ser Met Glu Ser Tyr Glu Val Ser Pro Phe

```
                  20                            25
30

Thr Thr Arg Arg Asn Ala Asn Thr Phe Ile Ser Pro Gln Gln Arg
           35                             40                        45


<210>  22
<211>  18
<212>  PRT
<213>  Homo sapiens

<400>  22

Met Lys Ser Gln Glu Ser Asp Glu Ala Ile Lys Val Ile Pro Val Ala
1                        5                            10
15


Gln Arg



<210>  23
<211>  17
<212>  PRT
<213>  Homo sapiens

<400>  23

Ile Lys Ala Glu Asp Ser Glu Glu Asn Gly Val Phe Lys Tyr Arg Pro
1                     5                              10
15


Arg



<210>  24
<211>  26
<212>  PRT
<213>  Homo sapiens

<400>  24

Cys Ala Gly Cys Cys Asn Asp Glu Ala Leu Glu Cys Val Pro Thr Ser
1                        5                                10
15


Glu Ser Asn Val Thr Met Gln Ile Met Arg
                   20                         25
```